# EUROPÄISCHE PATENTSCHRIFT

(11) **EP 3 458 119 B1**
(45) Veröffentlichungstag und Bekanntmachung des Hinweises auf die Patenterteilung: **27.01.2021**
(21) Anmeldenummer: 17734226.8
(22) Anmeldetag: 12.05.2017
(51) Int. Cl.: A61L 27/22

(54) **VERFAHREN ZUR BILDUNG EINES FUNKTIONELLEN NETZWERKES VON HUMANEN NEURONALEN UND GLIALEN ZELLEN**
METHOD FOR FORMING A FUNCTIONAL NETWORK OF HUMAN NEURONAL AND GLIAL CELLS
PROCÉDÉ POUR FORMER UN RÉSEAU FONCTIONNEL DE CELLULES NEURONALES ET GLIALES HUMAINES

(30) Priorität: 17.05.2016 DE 102016109068
(43) Veröffentlichungstag der Anmeldung: 27.03.2019
(73) Patentinhaber: Leibniz-Institut für Polymerforschung Dresden e.V., 01069 Dresden (DE); Deutsches Zentrum für Neurodegenerative Erkrankungen e.V., 53127 Bonn (DE)
(72) Erfinder: PAPADIMITRIOU, Christos, Southbank VIC 3006 (AU); KIZIL, Caghan, 01097 Dresden (DE); FREUDENBERG, Uwe, 01219 Dresden (DE); WERNER, Carsten, 01324 Dresden (DE)
(74) Vertreter: Sperling, Thomas
(86) Internationale Anmeldenummer: PCT/DE2017/100408
(87) Internationale Veröffentlichungsnummer: WO 2017/198258

(56) Entgegenhaltungen:
- WO-A1-2010/060485
- TSURKAN M.V. ET AL.: "Defined Polymer-Peptide Conjugates to Form Cell-Instructive starPEG-Heparin Matrices In Situ", ADVANCED MATERIALS, 2013, XP055393137, in der Anmeldung erwähnt
- SE HOON CHOI ET AL: "A three-dimensional human neural cell culture model of Alzheimer's disease", NATURE, Bd. 515, Nr. 7526, 12. Oktober 2014 (2014-10-12), Seiten 274-278, XP055394768, ISSN: 0028-0836, DOI: 10.1038/nature13800 in der Anmeldung erwähnt
- DAWEI ZHANG ET AL: "A 3D Alzheimer's disease culture model and the induction of P21-activated kinase mediated sensing in iPSC derived neurons", BIOMATERIALS., Bd. 35, Nr. 5, 1. Februar 2014 (2014-02-01), Seiten 1420-1428, XP055394758, GB ISSN: 0142-9612, DOI: 10.1016/j.biomaterials.2013.11.028 in der Anmeldung erwähnt

## Beschreibung

Die Erfindung betrifft ein Verfahren zur Bildung eines funktionellen Netzwerkes von humanen neuronalen und glialen Zellen, im Folgenden auch neuronales Netzwerk genannt. Das Verfahren kann für das Monitoring der Bildung des neuronalen Netzwerks und dabei insbesondere zur Modellierung von Krankheiten, die sich auf die Bildung von Neuronen und/oder neuronalen Netzwerken im menschlichen Gehirn auswirken, angewendet werden.

Eine aktuelle Studie von D.Y. Kim et. al. A three-dimensional human neural cell culture model of Alzheimer's disease, Nature 2014, 515, 274-278**,** beschreibt gentechnisch modifizierte menschliche Zellen, die in Matrigel® der Firma BD Biosciences eingebettet wurden, um eine dreidimensionale Dünnschichtkultur mit einer Gesamt-Axialebene von nicht mehr als 0,3 mm bereitzustellen. Die differenzierten Neuronen waren lebensfähig und funktional für 4 bis 12 Wochen. In einer ähnlichen Studie von Dawai Zhang et al., A 3D Alzheimer's disease culture model and the induction of P21-activated kinase mediated sensing in iPSC derived neurons, Biomaterials 2014 Feb; 35 (5), 1420-1428**,** wurde von der Verwendung der aus menschlichen induzierten pluripotenten Stammzellen (iPSCs) abgeleiteten neuroepithelialen Stammzelllinie (It-NES)-Linie AF22 und ihrer Differenzierung in neuronale Linien berichtet. Darüber hinaus wurde ein Hydrogel verwendet, das aus PuraMatrix® der Firma BD Biosciences hergestellt und mit 10 µg/mL Laminin modifiziert wurde. Dieses Kultursystem basiert auf einem durch selbstassemblierende Arginine-Alanine-Aspartic Acid-Alanine (RADA-single letter code)-Peptiden durch nichtkovalente Wechselwirkungen vernetztem Hydrogelsystem. Die mechanischen Eigenschaften des Hydrogeles sind aufgrund der relativ schwachen nichtkovalenten Wechselwirkungen nur begrenzt einstellbar und es bestehen auch keine für spezifische Enzyme sensitiven Spaltstellen. Aufgrund der Nichtabspaltbarkeit von Zellen in der BD-PuraMatrix® bietet dieses Kultursystem keine zellresponsive Mikroumgebung. Ein notwendiger Hydrogelumbau für das Wachstum von eingebetten Zellen kann nur durch die unspezifische Degradation der Peptide und damit durch Abbau der gesamten Hydrogelmatrix erfolgen. Entsprechend kann gemäß des bei der Studie vpn Zhang et al. eine 3D-Kultivierung der Zellen nur über sehr kurze Zeiträume, zum Beispiel 2-4 Tage, erfolgen. In der Schrift von Zhang wird explizit darauf hingewiesen, dass eine "Langzeitkultivierung aufgrund der geringen Steifigkeit des Materials technisch schwierig ist". Außerdem wird durch das Einmischen von aus tierischen Quellen gewonnenes Laminin ein schlecht definiertes, der Gefahr von Verunreinigungen beziehungsweise chargenabhängigen Schwankungen in der Produktqualität unterliegendem Protein Bestandteil des Assays, der starke Zweifel an der Reproduzierbarkeit der Präparation (Verfahrens) aufkommen lässt. Darüber hinaus können durch die lange Gelierungszeit (Gelbildungszeit) des Materials (20-30 Minuten) lokale Entmischungseffekte und Inhomogenitäten der eingebetten Zellen auftreten.

Aus S. Koutsopoulos et. al., Long-term three-dimensional neural tissue cultures in functionalized self-assembling peptide hydrogels, Matrigel and Collagen I, Acta Biomater. 2013, Feb; 9 (2); 5162-5169**,** ist die Herstellung eines nicht-zellresponsiven, selbstorganisierenden Peptids ähnlich wie PuraMatrix® bekannt, wobei neuronale Maus-Zellen kultiviert wurden. Die Studie von J.Y. Sang, Simple and Novel Three Dimensional Neuronal Cell Culture Using a Micro Mesh Scaffold, Exp Neurobiol. 2011 Jun; 20 (2); 110-115**,** beschreibt eine dreidimensionale neuronale Kultur unter Nutzung synthetisierter Nylonfasern als Gerüst. Neuronale Zellen werden mit Agarose gemischt und dann oben auf das Nylon-Gewebe plattiert. Eine derartige Technik bietet eine künstliche Umgebung, in der Zellen in eine zellresponsive Umgebung eingekapselt sind, welche nicht geeignet ist, das In-vivo-Milieu eines sich entwickelnden Gehirns nachzubilden.

In der US 6 306 922 A und der US 6 602 975 A ist ein photopolymerisiertes Hydrogel beschrieben, welches biologisch abbaubar ist. Polymerisationen bei Wellenlängen nahe dem UV-Spektrum kann jedoch in Zellkultursystemen aufgrund der Bildung von freien Radikalen Zelltod und DNA-Mutationen verursachen. Durch UV-Wellen hervorgerufene Zellschäden sind, im erfindungsgemäßen System nicht zu erwarten, da die Polymerisation unter normale Laborbedingungen und ohne UV-Licht durchgeführt wird. Darüber hinaus macht der Verzicht auf eine UV-induzierte Photopolymerisation die Anwendungen des hier beschriebenen Hydrogelsystems außerhalb hochspezialisierter Laboratorien wesentlich einfacher, da keine spezielle Ausrüstung erforderlich ist, um die Polymerisation herbei zu führen.

Die Aufgabe der Erfindung besteht darin, ein im Vergleich zu den oben genannten Vorveröffentlichungen wesentlich verbessertes modulares In-vitro-System beziehungsweise Verfahren mit Zellen humaner Herkunft zu entwickeln, welches funktionelle neuronale Netzwerke in einer dreidimensionalen Matrix ausbildet. Das wichtigste Bewertungskriterium für ein solches System ist die Qualität des neuronalen Netzwerks, das die In-vivo-Situation in neuronalem Gewebe des zentralen Nervensystems so weit wie möglich nachbilden soll. Zudem soll das System einfach zu handhaben sein und eine sicherere Perspektive für Anwendungen ohne hochspezialisierte Laboratorien bieten, zum Beispiel auch bei Transplantationsverfahren.

Die Aufgabe wird in Form eines Verfahrens zur Bildung eines funktionellen Netzwerkes von humanen neuronalen und glialen Zellen nach Anspruch 1 gelöst. Weiterbildungen sind in den abhängigen Ansprüchen angegeben. Weitere Ansprüche beziehen sich auf Anwendungen des Verfahrens.

Erfindungsgemäß werden bei dem Verfahren zur Bildung eines funktionellen Netzwerkes von humanen neuronalen und glialen Zellen die Zellen in ein synthetisches Hydrogelsystem mit den Komponenten Polyethylenglykol (PEG) und Heparin eingebracht und darin kultiviert. Dabei erfolgt das Einbringen der Zellen in das PEG-Heparin-Hydrogelsystem zusammen mit einer der Gelkomponenten, entweder PEG oder Heparin, mit der die Zellen zuvor vermischt wurden, so dass sich die Zellen während der Polymerisation des dreidimensionalen Hydrogels bereits im Hydrogelsystem befinden.

Vorteilhafterweise werden bei dem Verfahren die humanen neuronalen Zellen mit glialen Zellen co-kultiviert, wobei die humanen neuronalen Zellen humane neuronale Stamm- und Vorläuferzellen sind oder einer humanen immortalisierten neuronalen Vorläuferzelllinie entstammen oder primäre humane, aus dem Mittelhirn gewonnene neuronale Vorläuferzellen sind.

Gemäß einer vorteilhaften Ausführungsform sind die humanen neuronalen Zellen humane neuronale Stamm- und Vorläuferzellen aus induzierten pluripotenten Stammzellen (iPSCs) oder sind aus primären humanen kortikalen Zellen abgeleitet.

Die Funktionalität des gebildeten Netzwerkes ist insbesondere durch die Expression reifer neuronaler kortikaler Marker, die Ansprechbarkeit auf Neurotransmitter, beispielsweise in Form von Calciumeinstrom, und durch elektrophysiologische Aktivität beschreibbar.

Gemäß einer bevorzugten Ausführungsvariante der Erfindung ist das PEG-Heparin-Hydrogelsystem ein mehrarmiges Polyethylenglykol (Stern-PEG) enthaltendes Stern-PEG-Heparin-Hydrogelsystem, das über enzymatisch spaltbare Peptidsequenzen, vorzugsweise durch Matrix-Metalloproteinasen spaltbare Peptidsequenzen (MMP-Peptide), vernetzt wird, wodurch das Stern-PEG-Heparin-Hydrogelsystem spaltbar und lokal umbaubar ist. Besonders bevorzugt wird als mehrarmiges Polyethylenglykol das vierarmige Polyethylenglykol (vierarmiges Stern-PEG).

In einer Ausführungsvariante wird die Hydrogelmatrix des Hydrogels durch eine kovalente Vernetzung eines thiolterminierten Stern-PEG-Peptid-Konjugats und eines durch Maleimid, vorzugsweise durch 4-6 Maleimidgruppen, funktionalisierten Heparins gebildet. Dabei erfolgt die Vernetzung der Hydrogelmatrix über eine Michael-Addition.

Alternativ wird die Hydrogelmatrix des Stern-PEG-Heparin-Hydrogelsystems nichtkovalent aus Heparin und einem kovalenten Stern-PEG-Peptid-Konjugat durch Selbstorganisation gebildet. Dabei umfasst das Stern-PEG-Peptid-Konjugat Konjugate von zwei oder mehr Peptiden, die an eine Polymerkette gekoppelt sind. Die Peptidsequenz enthält ein sich wiederholendes Dipeptid-Motiv (BA)ₙ, wobei B eine Aminosäure mit einer positiv geladenen Seitenkette, A Alanin und n eine Zahl von 5 bis 20, vorzugsweise 5 oder 7, ist.

Gemäß einer besonders vorteilhaften Ausführungsform der Erfindung weist das Hydrogel variierbare mechanische Eigenschaften, charakterisiert durch das Speichermodul auf. Vorzugsweise ist das Speichermodul in einem Bereich von 300-600 Pascal variierbar. Der Bereich des Speichermodules kann beispielsweise durch Einstellung des Mischungsverhältnisses der beiden Materialkomponenten, das heißt durch Variation des Vernetzungsgrades (gleichbedeutend mit der Variation des molaren Verhältnisses von PEG zu Heparin), oder durch Variation des Feststoffgehaltes der Materialkomponenten, das heißt der Konzentration der polymeren Ausgangsstoffe, variiert und vorzugsweise mittels oszillatorischer Rheometrie bestimmt werden.

In einer bevorzugten Ausführungsform der Erfindung wird das PEG-Heparin-Hydrogelsystem mit Signalmolekülen und/oder mit von Proteinen der extrazellulären Matrix (EZM) abgeleiteten funktionellen Peptideinheiten, vorzugsweise Adhäsionspeptiden, modifiziert.

Gemäß einer weiteren Ausgestaltung der Erfindung erfolgt eine Co-Kultivierung der humanen neuronalen und glialen Zellen zusammen mit humanen mesenchymalen Stromazellen und Endothelzellen, die co-lokalisiert mit den humanen neuronalen und glialen Zellen vorliegen.

Im dem Verfahren können vorteilhafterweise nicht genetisch modifizierte humane neuronale Stammzellen verwendet werden. Die Zellen reifen natürlich im Hydrogel, vorzugsweise einem Stern-PEG-Heparin-Hydrogel, unter Bildung von vollständig differenzierten neuronalen Subtypen, welche positiv für neuronale Markerpoteine wie CTIP2+, SATB2 + und TAU+ sind. Darüber hinaus können die Kulturen bei Anwendung eines PEG-Heparin-Hydrogels für mehr als 10 Wochen überleben.

Im erfindungsgemäßen Verfahren werden durch die schnelle Hydrogelbildung, das heißt 30 Sekunden bis maximal 2 Minuten, nachteilige lokale Entmischungseffekte und Inhomogenitäten vermieden Die schnelle, robuste Hydrogelbildung ist ein wesentlicher Vorteil des erfindungsgemäßen Verfahrens.

Ein weiterer Aspekt der Erfindung betrifft ein humanes, neuronales, dreidimensionales funktionales Netzwerk, das nach dem erfindungsgemäßen Verfahren erhältlich ist. Durch Anwendung des Verfahrens konnten sich einzelne Neuronen in einem dreidimensionalen Netzwerk verknüpfen und zeigten physiologisch relevante Zellfunktionen. In einem Volumen von 0.18 mm³ entwickelten sich beispielsweise mehr als 200 Teilnetzwerke, die wiederum untereinander verknüpft waren. Jedes Teilnetzwerk hatte dabei eine Gesamtzahl von mehr als 12000 Verzweigungen. Im erfindungsgemäßen dreidimensionalen Zellkultursystem konnten eine starke Netzwerkbildung und neuronale Verzweigung mit ausdifferenzierten Neuronen und co-lokalisierten gliale Zellen verschiedenen Typs nachgewiesen werden, wie später in den Ausführungsbeispielen im Detail beschrieben.

Das erfindungsgemäße Verfahren unter Anwendung des Hydrogelsystems ermöglicht
- die Nachbildung der humanen dreidimensionalen Netzwerkstruktur und der Funktionalität des humanen neuronalen Netzwerks, insbesondere hinsichtlich der Morphologie, des Zelltyps und der Differenzierung,
- die Gewährleistung einer Stabilität der Zellkulturen, so dass die Zellkulturen über eine längere Kultivierungsdauer erhalten bleiben und insbesondere während der Langzeitanalysen stabil bleiben,
- die genaue Einstellung beziehungsweise Abstimmung der Hydrogelmatrix, der biomolekularen Zusammensetzung und der physikalischen Eigenschaften, wie des Speichermoduls, so dass verschiedene exogene zellinstruktive Signale und Signalstoffe, zum Beispiel lösliche Faktoren, Komponenten der extrazellulären Matrix und mechanische Eigenschaften, für die neuronale Entwicklung und für die Modellierung von Krankheiten getestet werden können,
- eine Reaktion des gebildeten neuronalen Netzwerks auf Wirkstoffe ähnlich wie unter In-vivo-Bedingungen und damit die Eröffnung einer Möglichkeit, kostenintensive Tierversuchsmodelle zu ersetzen und darüber hinaus auch eine zuverlässigere Testmethode therapeutische Wirkstoffe bereitzustellen,
- die Bestimmung der elektrophysiologischen Aktivität und MembrankanalAktivität der neuronalen Zellen,
- die Analyse einzelner Zellen,, zum Beispiel mit hochauflösenden Bildern und Aufnahmetechniken, um die Signalleitungen und neuronalen Schaltkreise in einem dreidimensionalen neuronalen Netzwerk untersuchen zu können,
- die Analyse in Echtzeit insbesondere von Entwicklungsprozessen des neuronalen Netzwerkes,
- Array-Techniken, wie zum Beispiel Druck, und zonale Heterogenität, um Organ-Mimetika zu entwickeln,
- die Anwendbarkeit für personalisierte Medizin unter Anwendung von vom Patienten stammenden Zellen,
- eine Co-Kultivierung mit Endothelzellen, um die Wechselwirkung der neuronalen Netzwerkbildung und der Gefäßbildung unter In-vivo-ähnlichen Bedingungen zu untersuchen,
- die Entnahme von Einzelzellen, die für Einzelzell-Assays, die In-vitro-Zellexpansion und für Transplantationszwecke notwendig ist.
- eine Transplantation eines nichttoxischen und bioabbaubaren Materials;
- eine Langzeitkultivierung von mehr als zehn Wochen;
- eine Quantifizierung von Änderungen und des Fortschritts von neuronalen Netzwerken und der Zellanzahl.

Ein weiterer Aspekt der Erfindung betrifft die Anwendung des erfindungsgemäßen Verfahrens zum Monitoring der Bildung des neuronalen Netzwerkes. So ist insbesondere aufgrund der Transparenz der Zellkultur im dreidimensionalen Hydrogelsystem ein Echtzeit-Monitoring der Bildung des neuronalen Netzwerks möglich.

Das Monitoring der Netzwerkbildung unter Anwendung des erfindungsgemäßen Verfahrens ermöglicht eine quantitative Analyse des Zellwachstums, der Länge, der Anzahl und Dichte von Verzweigungen und/oder der Konnektivität und/oder der elektrophysiologischen Aktivität der neuronalen Zellen innerhalb des neuronalen Netzwerkes.

Damit ist auch die Modellierung von Krankheiten, die sich auf die Bildung von Neuronen und neuronalen Netzwerken im menschlichen Gehirn auswirken, von Entwicklungsstörungen des Nervensystems oder von neurodegenerativen Erkrankungen, möglich.

Eine besonders vorteilhafte Anwendung des Verfahrens besteht dabei in der Modellierung der durch krankheitsrelevante Proteinaggregate, zum Beispiel Amyloid-β-42, verursachten Neurotoxizität und/oder Veränderung der neuronalen Stammzellplastizität.

Das Monitoring der Netzwerkbildung unter Anwendung des erfindungsgemäßen Verfahrens eignet sich auch für Test von Molekülen und/oder Wirkstoffen beziehungsweise Medikamenten, welche die neuronale Aktivität und/oder Netzwerkbildung beeinflussen.

Weitere Einzelheiten, Merkmale und Vorteile von Ausgestaltungen der Erfindung ergeben sich aus der nachfolgenden Beschreibung von Ausführungsbeispielen mit Bezugnahme auf die zugehörigen Zeichnungen. Es zeigen:
- **Fig. 1:**: eine grafische Darstellung eines Ausführungsbeispiels zur Herstellung eines Hydrogels,
- **Fig. 2:**: mikroskopische Aufnahmen der Reifung des neuronalen Netzwerks über einen Zeitraum von drei Wochen,
- **Fig. 3:**: eine umfangreiche dreidimensionale Darstellung eines drei Wochen alten Gels, welches neuronale und gliale Netzwerke mit hoher Dichte enthält,
- **Fig. 4:**: die Immunreaktivität verkapselter Neuronen gegen Synaptophysin (Syn) und acetyliertes Tubulin (aTub),
- **Fig. 5:**: eine Messung der Gesamtfluoreszenzintensitäten vor und nach der Zugabe des Neurotransmitters Glutamat,
- **Fig. 6:**: die Dreifachfärbung eines Hydrogels mit Zellen im Alter von drei Wochen unter Anwendung von verschiedenen Markern/Farbstoffen,
- **Fig. 7:**: das Ergebnis der Inkubation der eingeschlossenen Zellen mit dem synthetischen Nucleosid Bromodesoxyuridin (BrdU) eine Woche nach dem Beginn der Zellkultur,
- **Fig. 8:**: die Bildung von verschiedenen neuronale Subtypen aus neu entstandenen Neuronen,
- **Fig. 9:**: im Hydrogel eingeschlossene und durch Proliferation neu entstandene neuronale Stamm- und Vorläuferzellen nach drei Wochen,
- **Fig. 10:**: die Expression von Neurofilament als zusätzlicher Beweis des ausgereiften Differenzierungszustands von humanen neuronalen Stamm- und Vorläuferzellen (NSPCs),
- **Fig. 11:**: eine grafische Darstellung der Herstellung des PEG-Heparin-Hydrogels zur Untersuchung des Effekts von Amyloid-β-42-Peptiden in primären humanen kortikalen Zellen (PHCC),
- **Fig. 12:**: die dreifache Immunfärbung des Hydrogels mit Antikörpern gegen acetyliertes Tubulin als neuronales zytoplasmatisches Markerprotein, mit Aß42 als Marker für Peptid-Aggregation und mit GFAP als cytoplasmatischem Marker für gliale Zellen,
- **Fig. 13:**: maximale Intensitätsprojektion der skelettierten verbundenen neuronalen Bahnen in Hydrogelen ohne Aß42 (A), mit intrazellulärem Aß42 (A) und mit extrazellulärem Aß42 (A"),
- **Fig. 14:**: zweifache Immunfärbung gegen Acetyliertes Tubulin (Acet. Tubulin, Abbildung A/B), GFAP (Abbildung A"/B") mit Antikörpern und Kernfärbung (DAPI, Abbildung A'/B') von humanen neuronalen Stamm- und Vorläuferzellen (NSPCs) in Hydrogelen mit abgeleitet von (A) induzierten pluripotenten Stammzellen (iPSCs) und (B) primären humanen kortikalen Zellen (PHCCs),
- **Fig. 15:**: ein Vergleich der maximalen Intensitätsprojektion der neuronalen Fortsätze humaner neuronaler Stamm- und Vorläuferzellen (NSPCs), abgeleitet von iPSCs (A) oder PHCCs (B) mittels mikroskopischer Aufnahmen und in quantitativer Auswertung,
- **Fig. 16:**: mikroskopische Vergleichsaufnahmen von Stern-PEG-HEP-Gelen und darin eingebetteten PHCCs zur Untersuchung des Interleukin-4-Effekts auf die Aß42-Toxizität,
- **Fig. 17:**: mikroskopische Aufnahmen von Matrigel- und Stern-PEG-Heparin-Hydrogelen mit eingebetteten PHCCs für einen Vergleich der glialen Zellpopulation (GFAP), der neuronalen Netzwerkbildung (Acet Tubulin) sowie der Stammzellpopulationen (SOX2) und der neuroplastischen Kapazität.

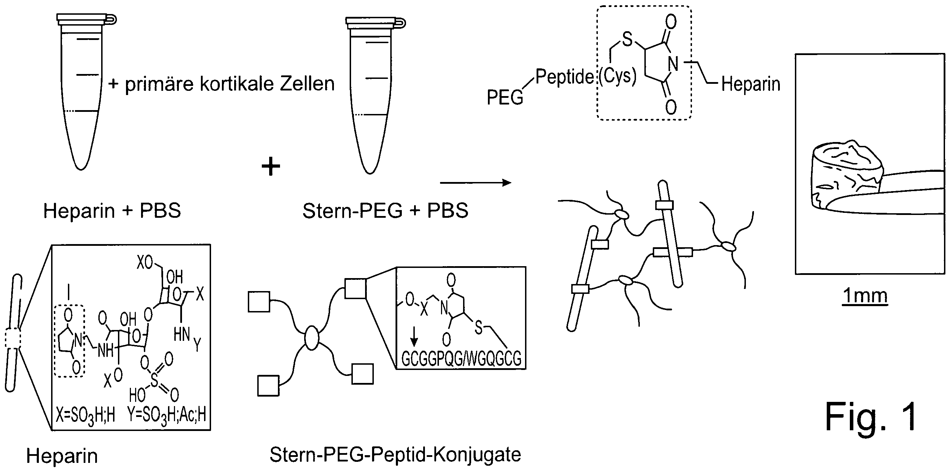

Die **Fig. 1** zeigt eine schematische grafische Darstellung eines Ausführungsbeispiels zur Herstellung eines Hydrogels. Gemäß dieser Darstellung werden primäre humane kortikale Zellen (PHCC) verwendet. Diese werden zunächst in phosphatgepufferter Salzlösung (PBS) mit Heparin zusammengebracht. Als weitere Komponente des Hydrogel neben dem Heparin dient in dieser Darstellung ein Konjugat aus einem vierarmigen Polyethylenglykol (Stern-PEG) und einem enzymatische spaltbaren Peptid, wobei das PEG an jedem Arm mit einem Peptidmolekül konjugiert ist. Diese Komponente wird in phosphatgepufferter Salzlösung (PBS) mit dem Heparin und den Zellen zusammengebracht. Die Hydrogelmatrix des Hydrogels wird durch eine kovalente Vernetzung des thiolterminierten (Cystein-Seitenkette, abgekürzt cys) Stern-PEG-Peptid-Konjugats und eines durch Maleimid funktionalisierten Heparins gebildet, wobei die Vernetzung der Hydrogelmatrix über eine Michael-Addition erfolgt.

Mehrere der folgenden Figuren zeigen jeweils mikroskopische Abbildungen, auf denen jeweils unten rechts ein Symbol erkennbar ist. Dieses Symbol stellt ein Auge dar, welches entweder von oben oder von der Seite auf ein zylindrisches Hydrogel schaut.

Das von oben schauende Auge stellt dar, dass das damit gekennzeichnete Bild ein Bild der maximalen Intensitätsprojektion einer Reihe von Bildern auf der z-Achse ist.

Das von der Seite schauende Auge stellt dar, dass das damit gekennzeichnete Bild ein Bild der maximalen Intensitätsprojektion einer Reihe von Bildern auf der x-Achse ist.

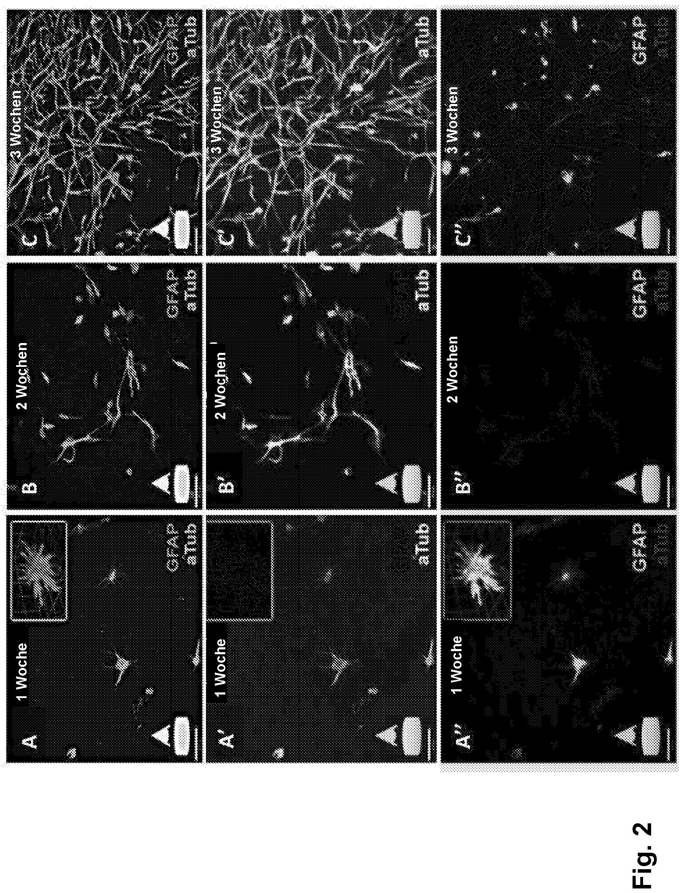

Die **Fig. 2** stellt die Reifung des neuronalen Netzwerks über einen Zeitraum von drei Wochen dar. Eine Färbung des zytoplasmatischen glialen Zellmarkers GFAP (von der englischen Bezeichnung "Glial fibrillary acidic protein" abgeleitet, saures Gliafaserprotein) markiert das Zytoplasma von glialen Zellen, das gegenüber den Neuronen eine Zunahme ergab. Das Zytoplasma von Neuronen ist mit acetyliertem Tubulin (aTub) gefärbt. Die Abbildungen A-A" zeigen jeweils ein typisches Bild der maximalen Intensitätsprojektion über die z-Achse des Zustands der eingeschlossenen Zellen nach einer Woche der Einbettung. Die Abbildungen B-B" zeigen jeweils ein typisches Bild der maximalen Intensitätsprojektion über die z-Achse des Zustands der eingeschlossenen Zellen nach 2 Wochen der Einbettung. Die Abbildungen C-C" zeigen jeweils ein typisches Bild der maximalen Intensitätsprojektion über die z-Achse des Zustands der eingeschlossenen Zelle nach drei Wochen der Einbettung. In der ersten Reihe ist die kombinatorische Färbung von GFAP und aTub-positiven Zellen zu sehen. In der zweiten und dritten Reihe ist zu sehen, dass die Zellen jeweils positiv auf die Marker aTub und GFAP reagieren.

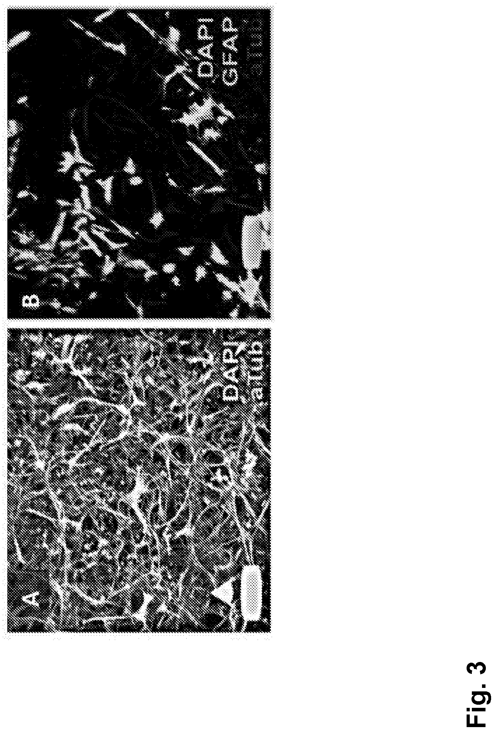

Die **Fig. 3** zeigt eine umfangreiche dreidimensionale Darstellung eines drei Wochen alten Gels, welches neuronale und gliale Netzwerke mit hoher Dichte enthält. Die glialen Zellen, die mit GFAP gefärbt sind, wirken eng mit Neuronen, welche mit acetyliertem Tubulin (aTub) gefärbt sind, zusammen, eine Erscheinung, die In-vivo-Situationen auftritt. Der Zellkernfarbstoff 4',6-Diamidin-2-phenylindol, abgekürzt DAPI, markiert die doppelsträngige Kern-DNA. Mit DAPI markierte Zellen waren zum Zeitpunkt, als die Proben zur Auswertung fixiert wurden, lebend. Die Abbildung A in Fig. 3 zeigt ein umfassendes Netzwerk von hinsichtlich aTub und DAPI zweifach positiven Neuronen. Die Abbildung B zeigt ein umfassendes Netzwerk von hinsichtlich GFAP und DAPI zweifach positiven glialen Zellen.

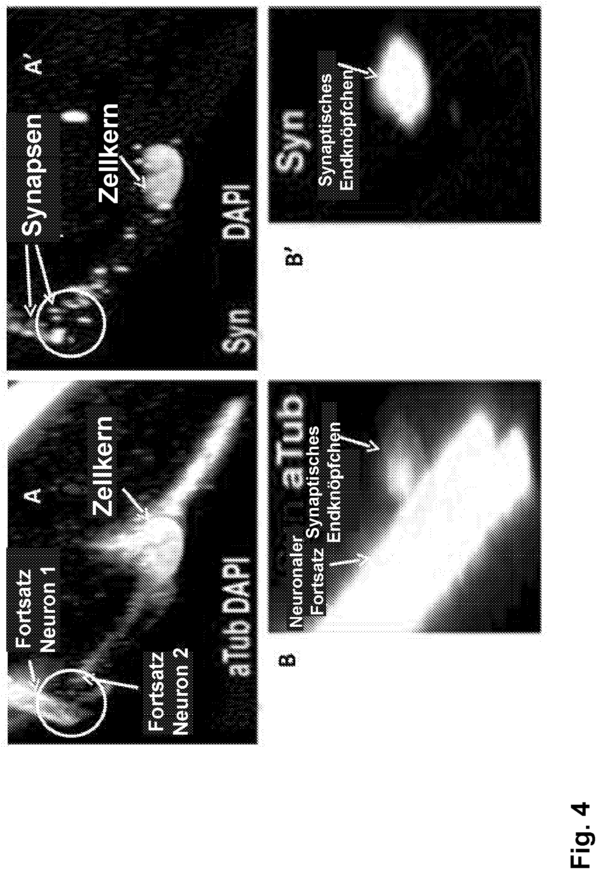

Die **Fig. 4** zeigt eine deutlich gehäufte Immunreaktivität in Zell-Kreuzungen und synaptischen Knöpfchen von eingebetteten Neuronen, wie sie auch in vivo in funktionellen Neuronen vorkommt. Dabei zeigen die Abbildungen A-A' und B-B': zweifach, mit acetyliertem Tubullin (aTub) und Synaptophysin (Syn), gefärbte Zellen. DAPI färbt die Zellkerne. Wie die Abbildung A zeigt, färbt aTub die Fortsätze der Neuronen 1 und 2, wobei die Neuronen mit den Pfeilen hervorgehoben sind, während der Kreis den Übergang der Neuronen 1 und 2 anzeigt. In der Abbildung A' erscheint die Synaptophysin (Syn)-Färbung als Mehrzahl von synaptischen Punkten an den Verbindungen der Neuronen 1 und 2, die in Abbildung A markiert sind. Die Abbildung B zeigt eine starke Vergrößerung eines neuronalen Fortsatzes, welcher an einem synaptischen Knöpfchen anliegt. Die Abbildung B' zeigt, wie das synaptische Knöpfchen der Abbildung B positiv auf Synaptophysin (Syn) reagiert.

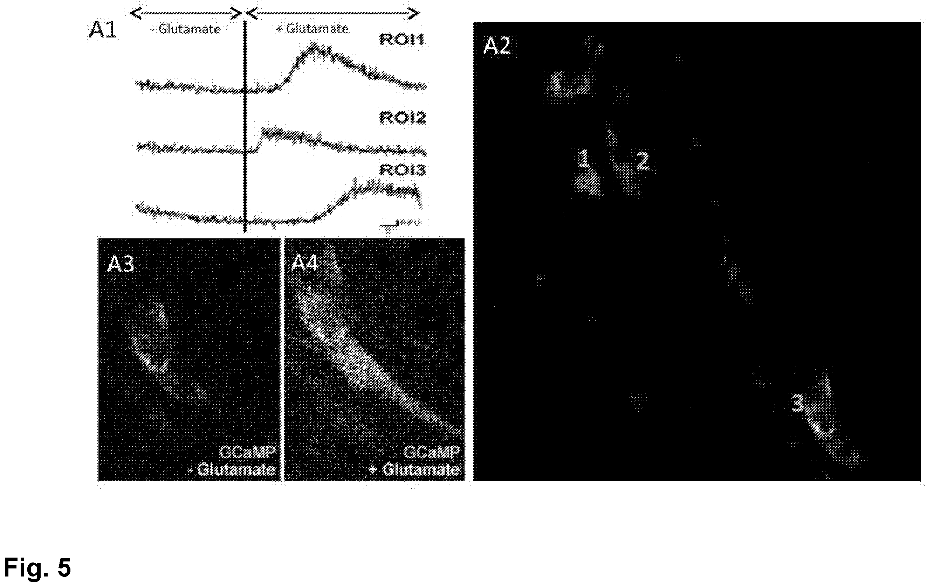

In der **Fig. 5** sind die Ergebnisse der Messung der Gesamtfluoreszenzintensitäten bei Zugabe des Neurotransmitters Glutamat dargestellt. Dabei zeigt die Abbildung A1 die Messung der Gesamtfluoreszenzintensitäten vor (-Glutamat) und nach (+Glutamat) der Zugabe des Neurotransmitters Glutamat, wie sie von den Zellen 1, 2 und 3 der Abbildung A (A2) emittiert worden sind. Die Zellen 1, 2, 3 der Abbildung A2 wurden mit dem Calciumsensor Gcampf6 transfiziert, der ein intensives Fluoreszenzsignal erzeugt, wenn die Zellen einen intrazellulären CalciumEinstrom als Antwort auf das zugesetzte Glutamat zeigen. Aus diesem Grund kann auf den Graphen von A1 ein erhöhtes Fluoreszenzsignal beobachtet werden, was bedeutet, dass die transfizierten Zellen in Gegenwart von Neurotransmittern reagieren, wie es auch in der In-vivo-Situation geschieht. Die Zellen in der Abbildung A2 sind vor der Zugabe des Neurotransmitters Glutamat in ein PEG-Heparin-Hydrogelsystem eingebettet. Die Abbildungen A3 und A4 zeigen eine starke Vergrößerung einer Gcampf6-transfizierten Zelle, die im PEG-Heparin-Hydrogelsystem eingebettet ist. Die Abbildung A3 zeigt die Zelle vor dem Zusatz von Glutamat. Die Abbildung A4 zeigt, dass nach dem Zusatz von Glutamat ein intensives Signal aufgrund des Einstroms von Calciumionen gemessen wird.

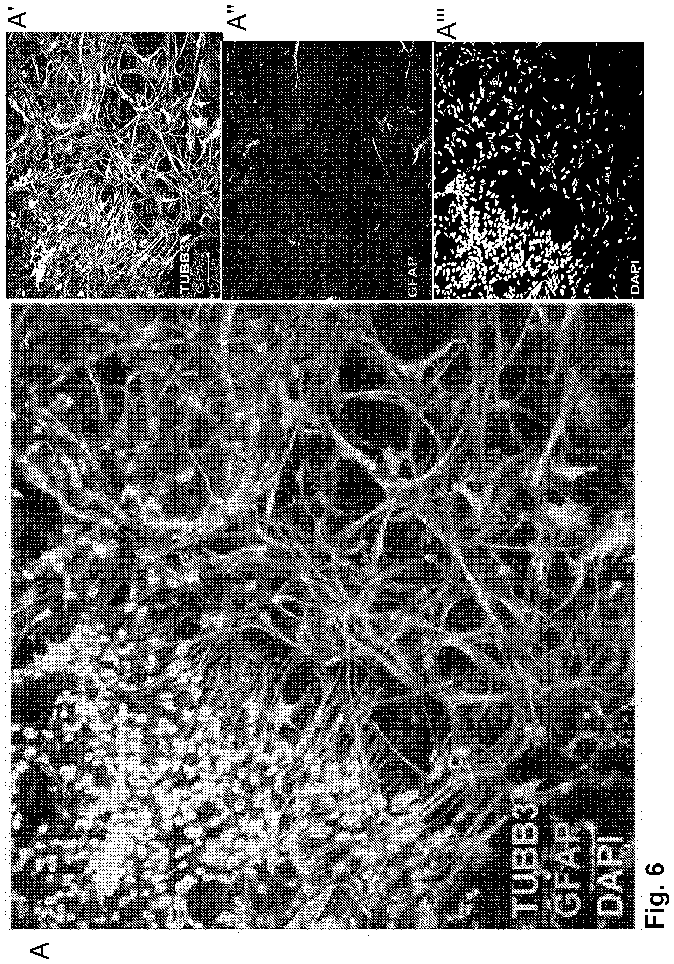

Die **Fig. 6** zeigt die Dreifachfärbung eines Hydrogels mit Zellen im Alter von drei Wochen, mit Immunoreaktivität gegen den neuronalen zytoplasmatischen Marker β-III-Tubulin (TUBB3), den zytoplasmatischen glialen Zellmarker GFAP und den DNA-Farbstoff 4',6-Diamidin-2-phenylindol (DAPI). Es sind verschiedene morphologische Eigenschaften der geclusterten Zellen erkennbar: deutlich ausgerichtete Zellfortsätze in der Abbildung oben links und ein gitterartiges neuronales Netzwerk gemischt mit glialen Zellen in der Mitte-links. Die Abbildungen A'-A'" zeigen Einzelaufnahmen der optischen Kanäle für TUBB3, GFAP, DAPI.

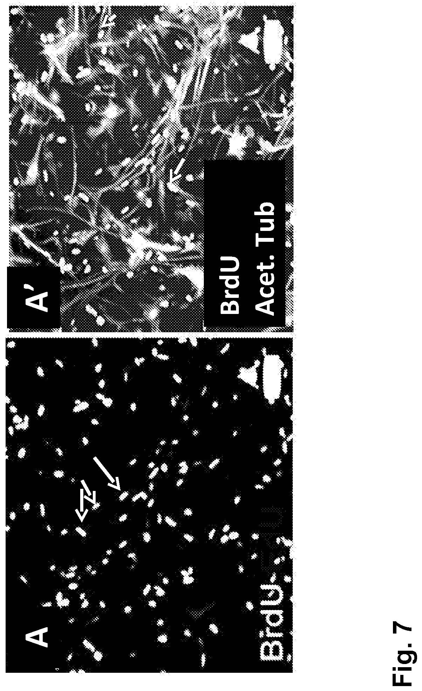

Eine Woche nach dem Start der Kultur der im PEG-Heparin-Gel eingeschlossenen Zellen wurden diese mit dem synthetischen Nucleosid Bromodesoxyuridin (BrdU) für 3 h inkubiert. Das Ergebnis ist in **Fig. 7** zu sehen, wobei Pfeile jeweils auf gefärbte Zellen hinweisen. Die Abbildung A zeigt mit anti-BrdU Antikörper gefärbte Zellen (Färbung des Zellkerns). Die Abbildung A' zeigt, dass durch anti-BrdU Antikörper angefärbte Zellen auch positiv für das zytoplasmatische neuronale Markerprotein acetyliertes Tubulin (aTub) sind. Die **Fig. 7** zeigt mit Hilfe der BrdU-Färbung, dass die im Hydrogel eingeschlossenen Zellen proliferieren und neuronale Identität haben (durch Acet. Tub. gefärbte Zellen).

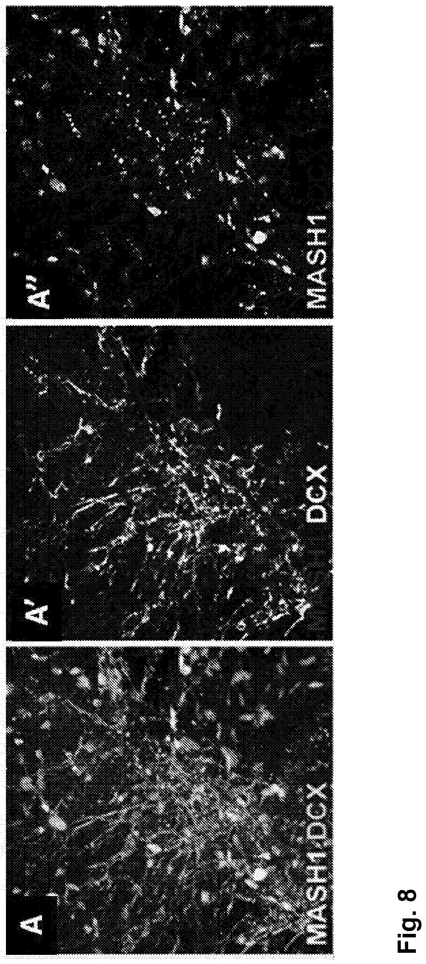

Die **Fig. 8** zeigt, dass aus den im Hydrogel eingebettete und darin neu entstandenen Neuronen verschiedene neuronale Subtypen entstehen, die denjenigen Zelltypen gleichen, die auch in vivo im Verlauf der neuronalen Zelldifferenzierung entstehen. Dabei zeigt die Abbildung A, dass Zellen im Hydrogelsystem zweifach positiv für die Marker für neuronale Vorläuferzellen MASH1, auch bekannt unter der englischen Bezeichnung "Achaete-scute family bHLH transcription factor 1", ASCL1, und Doublecortin/DCX sind. Die Abbildungen A' und A" zeigen die einzelnen optischen Kanäle für DCX und MASH1 Färbung.

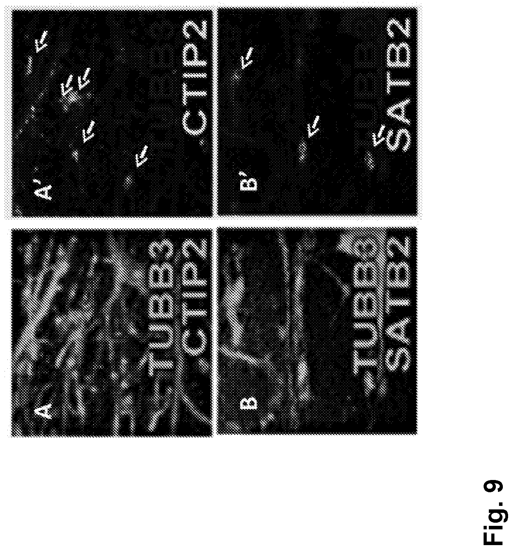

Die **Fig. 9** zeigt, dass im Hydrogel eingebettete und durch Proliferation neu entstandene neuronale Stamm- und Vorläuferzellen innerhalb von drei Wochen zu Zellen heranreifen, die Markerproteine für reife Kortikalneuronen exprimieren, zum Beispiel CTIP2 und SATB2.

Dabei zeigt die Abbildung A, dass die Zellen im Hydrogelsystem zweifach positiv für den nukleären kortikalen Marker CTIP2, auch bekannt unter der englischen Bezeichnung "B-cell CLL/lymphoma 11B", BCL11b, und für den neuronalen zytoplasmatischen Marker TUBB3 sind.

Dabei weisen in der Abbildung A' Pfeile auf CTIP2 positive Zellkerne der TUBB3 positiven Neurone hin. Die Abbildung B, zeigt, dass die Zellen zweifach positiv für den nukleären kortikalen Marker SATB2, dessen Bezeichnung eine Abkürzung der englischen Bezeichnung "Special AT-rich sequence-binding protein 2" darstellt, und den neuronalen zytoplasmatischen Marker TUBB3 sind. In Abbildung B' weisen Pfeile auf SATB2-positive Zellkerne der TUBB3-positiven Neurone hin.

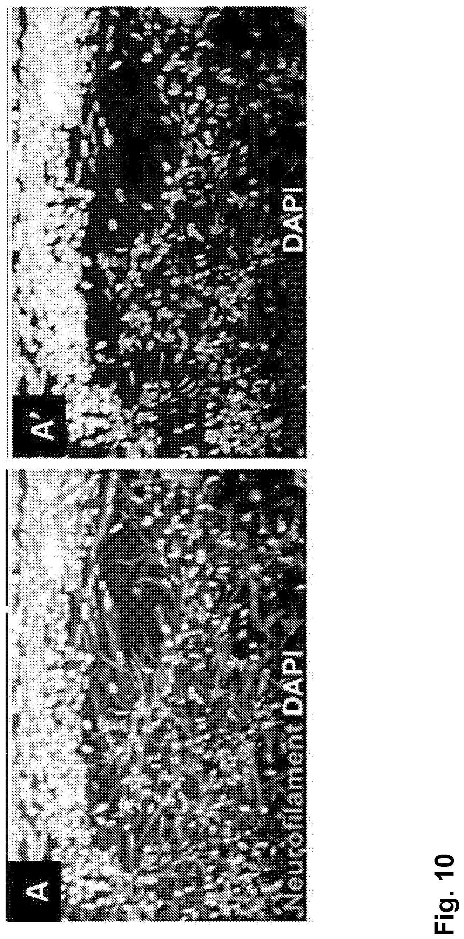

Aus der **Fig. 10** geht hervor, dass Zellen doppelt positiv für den Zellkernfarbstoff DAPI und das zytoplasmatische neuronale Protein Neurofilament, welches in reifen Neuronen exprimiert wird, sind. Dabei zeigt die Abbildung A' einen optischen Kanal für DAPI-Färbung aus Abbildung A. Die Expression von Neurofilament ist ein zusätzlicher Beweis des ausgereiften Differenzierungszustands der humanen neuronalen Stamm- und Vorläuferzellen (NSPCs) nach ihrer Einbettung und Reifung im PEG-Heparin-Hydrogel.

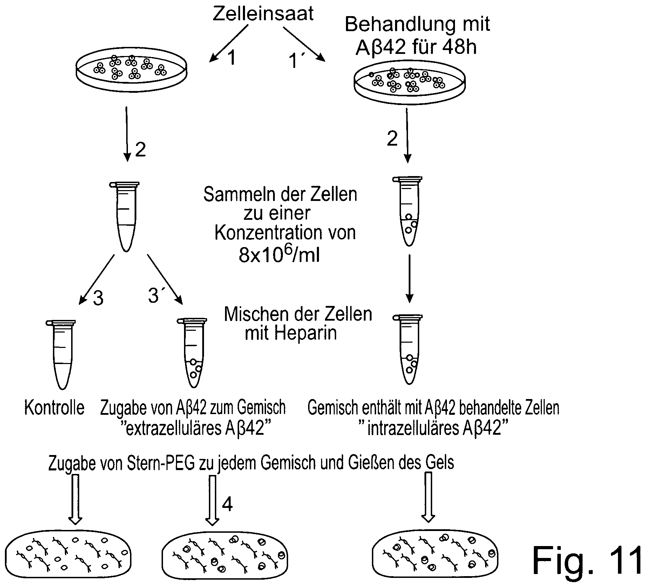

Die **Fig. 11** enthält eine grafische Darstellung der Herstellung des PEG-Heparin-Hydrogels zur Untersuchung des Effekts von Amyloid-β-42-Peptiden (Aβ42) in primären humanen kortikalen Zellen. Zellen der zweiten Passage wurden in einer Dichte von 5x10³ pro cm² in Schritt 1 in eine Petri-Schale platziert. Für die Zwecke des Aß42-Neurotoxizitätsmodells wurden ebenfalls Zellen der zweiten Passage in einer Dichte von 5x10³/cm² in einer Petrischale platziert und 48 Stunden lang mit 2 µM von Aß42 (Schritt 1') inkubiert.

Nach 48 Stunden wurden die Zellen geerntet und bei einer Konzentration von 8x10⁶ Zellen pro ml in phosphatgepufferter Salzlösung (PBS) in einem Schritt 2 resuspendiert. Dann wurde das gleiche Volumen Heparinlösung (45 µg/µl in PBS) hinzugegeben und beides in einem Schritt 3 zu einer Endkonzentration von 4x10⁶ Zellen/ml vermischt. Im Falle des Bestreichens der extrazellulären Umgebung der in das PEG-Heparin-Hydrogelsystem eingebetteten Zellen mit Aß42 wurde eine Mischung von Aß-42-Peptid in einer Konzentration von 40 µM in Schritt 3' hinzugegeben.

Um ein Gel zu gießen, wurden die Zelllösung, PBS und Heparin in Schritt 3 mit dem gleichen Volumen von Stern-PEG gemischt. Bei dieser Stufe hatten die Gele, die gemäß Schritt 3' gegossen wurden, eine Konzentration von extrazellulärem Aß42 von 20 µM. Die Konzentration der Zellen in allen Hydrogelen beträgt 2x10⁶ Zellen/ml. Die Reaktionen zur Gelbildung dauern zwei Minuten. Die Gele wurden nach dem Gießen in 24-Well-Kulturplatten platziert, wobei jedes Well ein Kulturmedium enthält. Um die Gele zu kultivieren und zu inkubieren, wurde ein Verhältnis von 5 % CO₂/95 % Luft bei 37 °C angewendet. Gele können bis zum gewünschten Zeitpunkt kultiviert werden.

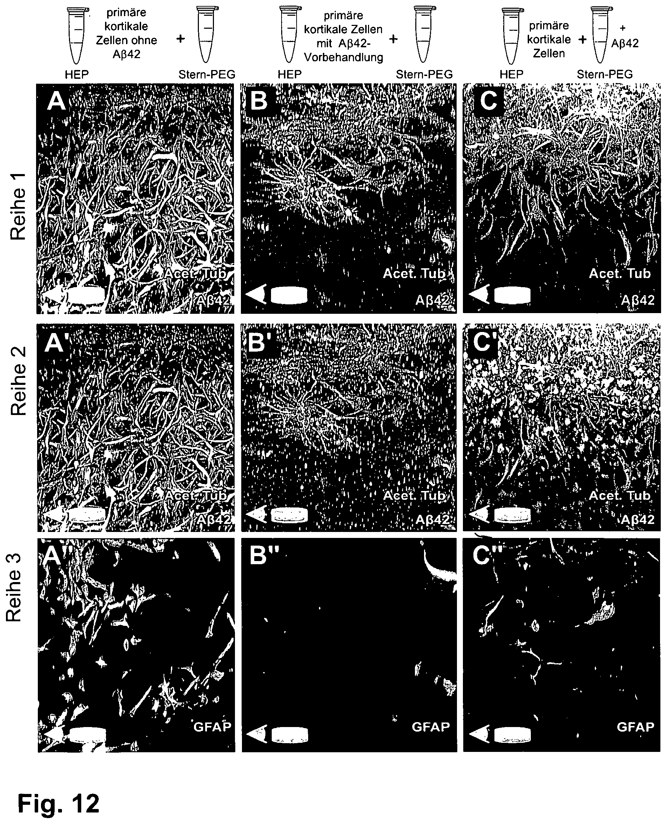

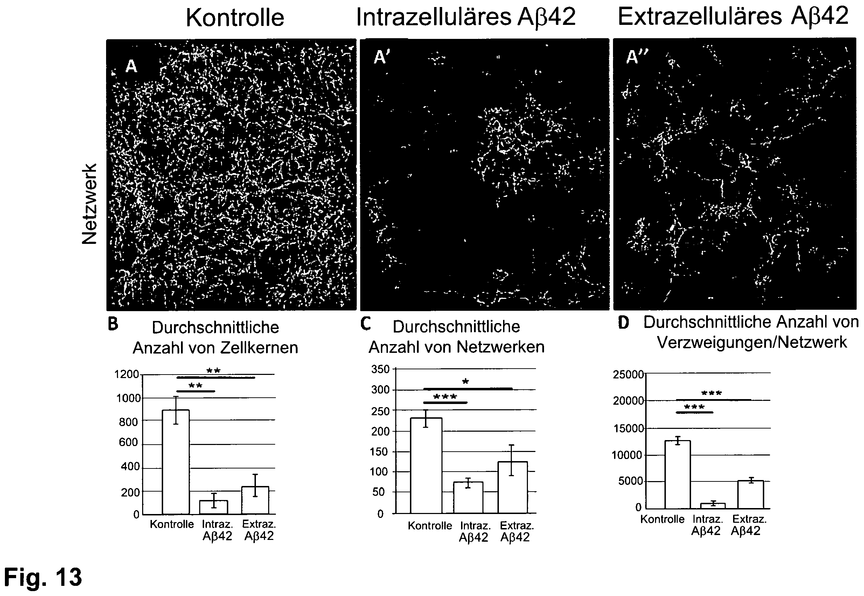

Die **Fig. 12** zeigt die dreifache Immunfärbung des Hydrogels mit Antikörpern gegen acetyliertes Tubulin als neuronales zytoplasmatisches Markerprotein, mit Aß42 als Marker für Peptid-Aggregation und mit GFAP als zytoplasmatischem Marker für gliale Zellen. Die **Fig. 12** zeigt Bilder der maximalen Intensitätsprojektion über die y-Achse von drei Wochen alten Gelen ohne Aß-42 in den Abbildungen A-A", mit intrazellularem Aß42 in den Abbildungen B-B", mit extrazellulärem Aß42 in den Abbildungen C-C". In der ersten Reihe der Abbildungen, das heißt den Abbildungen A-C ist der Kanal für die Färbung mit acetyliertem Tubulin dargestellt, der die gebildeten neuronalen Netzwerke hervorhebt. In der zweiten Reihe der Bilder (A'-C') ist das neuronale Netzwerk aus Reihe 1 dargestellt sowie die gebildeten Amyloid-Aggregate von Aß-42, die sich im ganzen Volumen des Gels ausgebreitet haben. Aussagekräftig ist der Verlust von neuronalen Netzwerken in Gegenwart von Aß42-Aggregaten, siehe Abbildungen B' und C' im Vergleich mit der Probe, die keine Aß-42-Aggregate enthält, vgl. Abbildung A'. In Reihe 3 ist der Kanal für die GFAP-Färbung dargestellt. Die Quantifizierung des zellulären Verlustes und des Verlustes des neuronalen Netzes durch Aß42-Aggregate ist in der nachfolgenden **Fig. 13** dargestellt.

Die **Fig. 13** zeigt die maximale Intensitätsprojektion der skelettierten verbundenen neuronalen Bahnen in Gelen ohne Aß42 (A), mit intrazellulärem Aβ42 (A') und mit extrazellulärem Aβ42 (A").

Die Bilder A-A" zeigen in allen Fällen den Kanal für aTub-positiven Zellen, das heißt im Fall der Kontrolle ohne Aβ42 (A), mit intrazellulärem Aβ42 (A') sowie mit extrazellulärem Aβ42 (A") der Fig. 12. Die Abbildung B der Fig. 13 zeigt die Quantifizierung der durchschnittlichen Zellzahl in Gelen ohne Aß42, mit intrazellulärem Aß42, mit extrazellulärem Aß42. Die Abbildung C zeigt die Quantifizierung der durchschnittlichen Anzahl von Netzwerken in Gelen ohne Aß42, mit intrazellulärem Aß42, mit extrazellulärem Aß42. Die Abbildung D zeigt die Quantifizierung der durchschnittlichen Anzahl von Verzweigungen pro Netzwerk in Hydrogelen ohne Aß42, mit intrazellulärem Aß42 und mit extrazellulärem Aß42.

Um eine Kulturbedingung zu schaffen, in welcher durch humane neurale Stamm- und Vorläufer Zellen (NSPCs) neuronale Netzwerke gebildet werden, wurden dreidimensionale PEG-Heparin-Hydrogele mit MMP-spaltbaren Stellen erzeugt. Diese Modifikation ermöglicht es den Zellen, ihre Umgebung neu zu strukturieren. Die Hydrogelsynthese ist bei Tsurkan M.V. et al. Defined Polymer-Peptide Conjugates to Form Cell-Instructive starPEG-Heparin Matrices In Situ. Advanced Materials (2013**)** beschrieben.

Wenn Zellen während der Polymerisationsstufe in diese PEG-Heparin-Hydrogele eingebracht werden, wird überraschenderweise beobachtet, dass bereits eine Woche nach Einbringung der Zellen das Gel spärlich verteilte GFAP-positive gliale Zellen mit einer 3D-verzweigten Morphologie enthält. Nach zwei Wochen der Zellkultur wird die Ausbreitung und Anordnung von Neuronen, die positiv für acetyliertes Tubulin sind, in Clustern beobachtet. Nach drei Wochen der Kultivierung zeigen die Zellkulturen umfangreiche, ausgefeilte Netzwerke von Neuronen mit eingestreuten gliale Zellen. Zudem sind die 3D-Kulturen der NSPCs durch den synaptischen Marker Synaptophysin anfärbbar, welcher an den neuronalen Knoten und Knotenpunkten akkumuliert, was auf reifere synaptische Verbindungen im Vergleich zu 2D-Kulturen hindeutet. Im Gegensatz hierzu kann in 2D-Kulturen mit neuronalen und glialen Zellen keine Synaptophysin Färbung in Clustern an Synapsen beobachtet werden. Neuronen in 3D-Hydrogele sind auch responsiv für Neurotransmitter, wie Glutamat, was durch die Erhöhung des intrazellulären Calciumspiegels, der durch die Transfektion von GCamP6f-exprimierenden Plasmiden mit einem CMV-Promotor angetriebenen Kalziumsensor bestimmt wird, nachgewiesen werden kann. Diese Ergebnisse zeigen, dass 3D-Kulturen der NSPCs in der Lage sind, ein umfassendes Netzwerk von Neuronen und gliale Zellen in einer dreidimensionalen Anordnung zu erzeugen. Ältere kortikale Subtyp-Marker wie CTIP2 und SATB2, proneurale Marker Mash1 und DCX und der ausgereifte neuronale Marker Neurofilament (Markerprotein für ausdifferenzierte Neuronen) werden auch in NSPC-Kulturen exprimiert, was darauf hindeutet, dass die in den 3D-Kulturen vorhandenen neuronalen Zellen sich unter den herrschenden Bedingungen zu reifen Neuronen entwickeln.

Es wurde auch Amyloid-β-42-Peptid verwendet, ein für die Alzheimererkrankung relevantes fehlgefaltetes Protein, um dessen Toxizität und dessen Effekte auf neuronale Netzwerke zu modellieren. Das erfindungsgemäße Verfahren unter Anwendung eines dreidimensionalen Hydrogelsystems zur Kultivierung kann auf diese Weise auch als Modell für neurodegenerative Krankheiten verwendet werden. Es konnte gezeigt werden, dass eine Amyloid-beta-42-Anhäufung die neuronale Netzwerkbildung und neuronale Konnektivität in vivo und in vitro beeinträchtigt. Zur Untersuchung, ob Amyloid-β-42 (Aß42) das neuronale Netzwerk beeinflusst, wurden Kulturen erzeugt, indem die Zellen vor dem Einbringen in das Hydrogelsystem mit Aß42 behandelt (intrazelluläres Aß42) oder die Hydrogele vor dem Einbringen der Zellen mit Aß42 inkubiert (extrazelluläres Aß42) wurden. Im Vergleich zu den Kontrollgelen, bei denen die Bildung von umfangreichen Netzwerkformen beobachtet werden konnte, beeinträchtigte intrazelluläres und extrazelluläres Aß42 die Netzwerkbildung signifikant. Im Vergleich zu den Kontrollgelen enthalten mit Aß42 behandelte Gele eine signifikant verringerte Anzahl an Zellen und Netzwerken. Überdies ist zu beobachten, dass auch dann, wenn in mit Aß42 behandelten Gelen einige Netzwerke gebildet werden, diese Netzwerke eine signifikant geringere Anzahl an Verzweigungen pro Netzwerk enthalten. Es wurde zudem herausgefunden, dass die Aß42-Behandlung ähnlich wie im menschlichen Gehirn zur Dystrophie von Axonen führte. Diese Ergebnisse zeigen, dass die 3D-Gele die menschliche Pathophysiologie von Aß42 rekapitulieren können, das einen toxischen Effekt auf die Bildung von neuronalen Netzwerken ausübt, ungeachtet der neurogenen Kapazität der neuronalen Stamm-oder Vorläuferzellen. Darüber hinaus kann das dreidimensionale Hydrogelsystem als eine praktische Screening-Plattform genutzt werden, um Verbindungen zu testen, welche die neurogene Fähigkeit von menschlichen Stammzellen und die Bildung von neuronalen Netzwerken auch in Gegenwart von Aß42 wiederherstellen könnten.

In Weiterbildung des Standes der Technik wird ein modulares und gut steuerbares Hydrogelmaterialsystem verwendet, welches es ermöglicht, unabhängig zellinstruktive Signale, die in der natürlichen Zellumgebung (der sogenannten extrazellulären Matrix (EZM)) vorkommen, insbesondere aber die physikalischen Netzwerkeigenschaften (Steifheit des Hydrogels in einem Bereich von 200 Pa bis zu 6 kPa), die Abbaubarkeit und die biomolekularen Zusammensetzung (Funktionalisierung mit Adhäsions- und Signalpeptiden, löslichen Zytokinen und Wachstumsfaktoren) zu modulieren, wie aus Tsurkan M.V. et al. Defined Polymer-Peptide Conjugates to Form Cell-Instructive starPEG-Heparin Matrices In Situ. Advanced Materials (2013**)** bekannt. Daher kann eine Vielzahl von Parametern in systematischer Weise (und unabhängig voneinander) in einer Reihe von Materialeigenschaften getestet werden. Darüber hinaus wird das Hydrogel unter milden, zellfreundlichen Bedingungen vernetzt, um eine hohe Lebensfähigkeit der Zellen zu ermöglichen. Darüber hinaus können die Zellen mit einer zonalen Heterogenität innerhalb der Matrix gedruckt werden, um zonal differenzierte Strukturen mit einer guten Lebensfähigkeit zu erzeugen.

Die verwendeten dreidimensionalen Kulturen enthalten neuronale Zellen, die positiv für Markerproteine reifer Neuronen sind, wie zum Beispiel CTIP2 und SATB2. Dies ist ein Indiz für in Kultur entstandene reife kortikale Neuronen, die einen Differenzierungsgrad der Zellen sehr ähnlich zu in vivo-Bedingungen zeigen. Des Weiteren konnte die elektrophysiologische Aktivität und Membrankanalaktivität in kultivierten Zellen in 3D gemessen werden, eine Funktion, die ebenfalls die in-vivo-artige Charakteristik des erfindungsgemäß verwendeten Systems zeigt.

Kulturen mit umfangreichen neuronalen Netzwerken können in drei Wochen erzeugt werden und können mindestens 10 Wochen überleben. Aufgrund der schnellen Erzeugung und der Kulturbedingungen können Hydrogel 3D-Kulturen für iPS-basierte personalisierte Medizin während jeder Gehirn-Erkrankung angewandt werden, um z. B. vor einer klinischen Behandlung die Wirkungen verschiedener Wirkstoffe auf Patientenzellen zu testen. Darüber hinaus erleichtert das erfindungsgemäße Verfahren die schnelle Expansion von glialen und neuronalen Vorläuferpopulationen und kann für zellbasierte Therapien, die eine große Anzahl an Zellen erfordern, angewandt werden.

Ein weiterer wesentlicher Vorteil des hier zum ersten Mal beschriebenen 3D-Hydrogel-Zellkultursystems ist die Transparenz des Gelmaterials, welches die Zellen umschließt. Für analytische Zwecke sind die 3D-Kulturen transparent und können für mikroskopische Echtzeit-Aufnahmen und andere Analysen, die eine gute Transparenz des Gewebes erfordern, verwendet werden. Dementsprechend ermöglichen die 3D-Kulturen quantitative Messungen der Netzwerkbildung und neuronaler Verzweigungen über optische und mikroskopische Methoden, wobei die Netzwerkbildung über den gesamten Zeitraum der Kultivierung beobachtet werden kann. Wie bereits erwähnt, ermöglicht das System auch die Messung elektrophysiologischer Aktivität und der Membrankanalaktivität der einzelnen Neuronen und der neuronalen Schaltkreise. Des Weiteren wurde ein Algorithmus entwickelt werden, um die zellulären Verbindungen in den Gelen zu verfolgen und die statistischen Ergebnisse quantitativ darzustellen.

Nach derzeitigem Kenntnisstand ist das bevorzugt verwendete Stern-PEG-Heparin-Kultursystem mit primären humanen kortikalen Zellen das einzige 3D-Kultursystem für neuronale Zellen, welches quantifizierbare und umfassende neuronale Netzwerke bereitstellt.

Im System von Kim et al. ist das neuronale Netzwerk im Vergleich zum durch das erfindungsgemäße Verfahren erhältliche viel größeren Neuronen-Netzwerk, welches sich über den gesamten Kulturraum erstreckt, den das erfindungsgemäß verwendete PEG-Heparin-Hydrogel anbietet, deutlich minderer Qualität. Im System nach Koutsopoulos S. et al. wurde eine niedrige Zellüberlebensfähigkeit festgestellt und auch die Qualität des Netzwerkes ist deutlich schlechter als das, was das zellresponsive Stern-PEG-Heparin-System anbietet.

Zudem basieren beide Systeme (Kim/Koutsopoulos et al.) auf BD-Matrigel, einer extrazellulären Matrix, extrahiert aus dem Engelbreth-Holm-Swarm (EHS)-Maus-Sarkom, einem Tumorgewebe. Neben einer für Matrigel bekannten, starken Schwankung von Charge zu Charge, welche die Reproduzierbarkeit von Ergebnissen verkompliziert, verändern die tumortypischen Signalstoffe und Zellkomponenten, die ein solches Produkt enthält, die normale molekulare Signalübermittlung an die Zellen. Daher ist ein solches Produkt für Untersuchungen zur Hirnentwicklung sowie für Transplantations- und/oder Untersuchungen von neurodegenerativer Prozesse ungeeignet, da sich die molekularen Prozesse in vivo stark von dem im Tumorgewebe vorhandenen Milieu unterscheiden. Das erfindungsgemäß verwendete Hydrogelsystem basiert auf einem synthetischen PEG und biologisch derivatisiertem, aber gereinigtem Heparin mit gut bekannten molekularen Eigenschaften wie Molekulargewichtsverteilung und Funktionalität, welche immer vor der Verwendung getestet werden. Deshalb zeigt das vorliegende System auch keine Reproduzierbarkeitsprobleme und auch keine immunogenen Reaktionen.

Dawai Zhang and Koutsopoulus et al. verwendeten zudem ein selbstorganisierendes Peptid-Hydrogel und ein Kollagen-Typ-I-Gel für die Kultivierung von neuronalen Zellen. Bei Kollagen treten - ähnlich wie beim Matrigel - bei verschiedenen Chargen Variationen auf. Bei allen anderen bisher verwendeten Hydrogelsystemen (zum Beispiel PuraMAtrix, selbstorganisierende Peptide, Collagen I und Matrigel) sind die physikalischen Eigenschaften eher undefiniert und stark variabel und können nicht unabhängig von der biomolekularen Zusammensetzung variiert werden. Dementsprechend erlauben die dabei verwendeten Materialien keine unabhängige Untersuchung des Einflusses von mechanischen und biomolekularen Reizen und leiden unter einer schlechten Reproduzierbarkeit.

Da diese verschiedenen Reize, die sich aus der Zusammensetzung und Anordnung der extrazellulären Matrix (EZM) ergeben, einen wesentlichen Parameter für die Beeinflussung der Stammzellenaktivität und die Gewebeneubildung darstellen, erlauben die aus dem Stand der Technik bekannten Systeme nicht, mechanische und biomolekulare Signale getrennt voneinander zu untersuchen. Vielmehr stellen In-Vitro-Tests aufgrund des komplexen Zusammenwirkens zwischen der Vielzahl an von der extrazellulären Matrix abgeleiteten Signalen und ihrer pleiotropen Wirkungen eine Herausforderung bei der Identifizierung der Funktion von exogenen Reizen auf die Gewebestrukturierung dar.

Als solche können die hier genutzten gut definierten und modular abstimmbaren PEG-Heparin-Hydrogele angewandt werden, um die mechanischen und biomolekularen Signale unabhängig voneinander zu modulieren, da die Zusammensetzung der Hydrogele unabhängig einstellbar ist (Tsurkan M.V. et al. Defined Polymer-Peptide Conjugates to Form Cell-Instructive starPEG-Heparin Matrices In Situ. Advanced Materials (2013**)).** Desweiteren sind die erfindungsgemäßen 3D-Hydrogelsysteme auch zellresponsiv, zum Beispiel durch MMP-spaltbare Stellen, was zum Beispiel von der Zelle ausgelöste Umbauprozesse und den Ersatz durch die eigene Matrix ermöglicht, um eine größere Ähnlichkeit mit den In-vivo-Verhältnissen im Hirngewebe zu erreichen. Den zuvor beschriebenen bekannten Verfahren für dreidimensionale neuronale Netzwerke fehlt eine definierte Zusammensetzung, welche die gezielte Modulation der mechanischen und biomolekularen Eigenschaften der 3D-Kultursystem sowie die zellabhängige Reorganisation der extrazellulären Matrix im 3D-Hydrogel erlaubt.

Deshalb könnten die verwendeten 3D-Zellkulturplattformen als ein vorteilhaftes Zellkultursystem dienen, um die Rolle der Matrixeigenschaften bei der Stammzellenaktivität und Differenzierung aufzuklären, unter der Voraussetzung, dass die Zellen dynamisch mit dem Hydrogelsystem zusammenwirken, um eine zellbedeckte extrazelluläre Matrix zu erzeugen. Zusätzlich können die verwendeten 3D-Hydrogelsysteme sowohl kovalent mit Adhäsions- oder Signalmolekülen als auch nicht kovalent mit Heparin-bindenden Signalmolekülen beschichtet werden. Insgesamt kann der Einfluss von multiplen zellinstruktiven exogenen Signalen systematisch mit Bezug auf die Zellproliferation von humane neuronalen Stamm- und/Vorläuferzellen und die neuronale Netzwerkbildung untersucht werden.

Viele 3D-Systeme, einschließlich Organoide, können keine in Größe und Form reproduzierbaren Strukturen bilden. Die erfindungsgemäßen 3D-Kulturen können für diese zwei Parameter angepasst und gezielt gesteuert werden und bieten somit wesentlich besser definierte Bedingungen für die 3D-Zellkultur.

Das bioabbaubare Hydrogel, welches in der US 6,306,922 A und der US 6,602,975 A beschrieben wurde, ist ein photopolymerisiertes Hydrogel. Das heißt, es benötigt für die Polymerisierung und Gelbildung unter spezifischen elektromagnetischen Bedingungen ein spezielles, ultraviolettes Licht (UV-Licht) emittierendes Gerät. UV-Licht führt zur Erzeugung von freien Radikalen an den eingebetteten Zellen und zur Induktion von apoptotischen Signalwegen, die zum Zelltod führen können und die Zellvitalität negativ beeinflussen. UV-Licht verursacht zudem DNA-Mutationen und Schädigungen der zellulären DNA der eingebetteten Zellen. Im Gegensatz dazu wird im hier beschriebenen innovativen 3D-Hydorgel System die Matrix bei Raumtemperatur und ohne den Einsatz von UV-Licht polymerisiert. Auf diese Weise entstehen keine DNA Schäden und Mutationen an den Zellen, so dass zelluläre Funktionen besser erhalten bleiben. Außerdem erlaubt der Verzicht auf eine UV-induzierte Polymerisation den Einsatz des 3D-Hydrogel Systems ohne den Einsatz teurer Geräte. Dieser Vorteil gestaltet das System nutzerfreundlich und damit ideal für die Anwendung außerhalb hochspezialisierter Laboratorien.

Darüber hinaus ist das vorliegende System das einzige, das unter Verwendung von Plasmiden die gezielte Gen-Fehlexpression ermöglicht, um eine funktionsfähige Version eines Gens zu überexprimieren (Verstärkung der Funktion) oder ein Gen herunterzuregeln, z. B. durch Verwendung von siRNAs (small interfering RNAs) oder nicht-funktioneller dominant-negativer Varianten eines Gens (Abschwächung oder Verlust der Funktion).

Es wurde die Nutzung eines Kalziumsensors (GcaMP) beschrieben, der durch ein Plasmid-Expressionssystem angetrieben wird. Das Fehlexpressionssystem beruht auf der Plasmidtransfektionsmethode, die auf die 3D-Gele abgestimmt wurde.

Verglichen zu früheren Berichten über die Modellierung der Alzheimer-Krankheit (AD) in 3D-Kulturen mit Matrigel Anwendung, erlauben PEG-Heparin-3D-Gele eine signifikant schnellere Entwicklung von Netzwerken, welches zum Beispiel für die Anwendung in Hochdurchsatz-Screening-Plattformen für Wirkstoffe einen Vorteil bietet.

Bisher konnte keine umfassende Netzwerkbildung in 3D-Gelsystemen mit menschlichen Neuronen gezeigt werden. Bisherige 3D-Kulturen nutzten schwimmende oder in Matrigel eingebettete Systeme und nutzen modifizierte Zellen.

Die Qualität der Netzwerkbildung konnte in früheren 3D-Kulturen nicht begutachtet werden, da die Ausbildung von Netzwerken nicht quantifiziert werden konnte. Die durch das erfindungsgemäße Verfahren erhältlichen dreidimensionalen Kulturen ermöglichen quantitative Messungen der Netzwerkbildung, zum Beispiel die Länge und Anzahl von Axonen und Neuriten, Anzahl von Verzweigungs- und Verknüpfungspunkten, und der neuronalen Verzweigung. Dies war bisher nicht möglich. Des Weiteren erlauben erfindungsgemäß verwendete Systeme Echtzeitaufnahmen und das Monitoring der eingebetteten Zellen während der Zellkulturperiode.

Die 3D-Kulturen für die reifen kortikalen Neuronen exprimieren kortikale Marker, wie zum Beispiel CTIP2 und SATB2.

In den kultivierten Zellen können in 3D die elektrophysiologische Aktivität und die Membrankanalaktivität gemessen werden, indem die Transfektion von Genen unter Anwendung von Plasmid-Vektoren genutzt wird. Bei früheren Studien mit 3D-Kulturen in Gerüsten oder als Organoide musste das Genom der Zellen manipuliert werden. Mit dem möglichen Transfektionsverfahren kann eine Fehlexpression ohne genetische Veränderung der Zellen selbst oder der Verwendung von Viren durchgeführt werden.

Kulturen mit ausgedehnten neuronalen Netzwerken können in 3 Wochen erzeugt werden und können für mehr als 16 Wochen am Leben gehalten werden. Aufgrund der schnellen Erzeugung und der Kulturbedingungen können die 3D-Kulturen auch für iPS-basierte personalisierte Medizin während einer Hirnerkrankungen verwendet werden, um die Auswirkungen verschiedener Wirkstoffe auf Patienten an eigenen Zellen des Patienten vor einer klinischen Behandlung zu testen.

Die Kulturen sind optisch transparent und können für Echtzeit-Aufnahmen und andere Analysen, die eine deutliche Sichtbarkeit der Gewebe erfordern, verwendet werden. Dies ist nicht der Fall für zuvor bekannte 3D-Gerüst-basierte oder Organoid-basierte Systeme.

Es besteht keine Notwendigkeit, die Zellen genetisch zu modifizieren, um 3D-Netzwerke zu bilden,

Das erfindungsgemäße Verfahren unter Verwendung eines Hydrogelsystems System ermöglicht die schnellste Expansion von glialen Zellen und neuronalen Vorläuferzell-Populationen und kann für zellbasierte Therapien, bei denen große Mengen an Zellen benötigt werden, verwendet werden.

### Technische Details

### Beispiel 1

In Beispiel 1 wurden primäre humane kortikale Zellen (PHCC) verwendet.

Die PHCC wurden aus der Großhirnrinde von Gewebespenden von Feten der 21. Schwangerschaftswoche isoliert, und wurden von der Firma ScienCell Research Laboratory (SRL, Katalog-Nummer 1800) in der ersten Passagein eingefrorenem Zustand bezogen.. Die Zellen wurden als negativ bezüglich HIV-1, HBV, HCV, Mycoplasma, Bakterien, Hefe und Pilzen zertifiziert. Die PHCC wurden in herkömmliche T75-Kolben oder 24-Well-Platten platziert und unter Anwendung von Astrocyt-Medium (SRL, Katalog-Nummer 1801), ergänzt durch 5 % fötales Rinderserum (SRL, Katalog-Nummer 0010), 1 % von Astrozytwachstumsmittel (SRL, Katalog-Nummer 1852) und 1% von Penicillin/Streptomycinlösung (SRL, Katalog-Nummer 0503) in einen Inkubator mit einer Atmosphäre von 5 % CO₂/95 % Luft bei 37 °C kultiviert.

PEG-Heparin-Hydrogele wurden hergestellt, wie bei Tsurkan et al., Advanced Materials 2013, vol. 25 (18) pp. 2606-2610**,** beschrieben, mit folgenden Änderungen: Es wurden PHCC unter Anwendung von Accutase® (Firma Invitrogen) als Zellablösungsmedium aus Kulturgefäßen entnommen. Nach der Zentrifugation für 10 min bei 12.000 Umdrehungen pro Minute wurden die Zellen bei einer Konzentration von 8x10⁶ Zellen pro ml in phosphatgepufferter Salzlösung (PBS) resuspendiert. Die polymeren Ausgangsstoffe (Prekursoren) für die Hydrogelherstellung bestanden, wie bei Tsurkan et al., Advanced Materials 2013, vol. 25 (18) pp. 2606-2610 beschrieben, aus Heparin, funktionalisiert mit sechs Maleimidgruppen (HEP-HM6) mit einem Molekulargewicht von 15.000 g/mol, und vierarmigem SternPEG, funktionalisiert mit enzymatisch spaltbaren Peptidsequenzen an jedem Arm mit einer Gesamtmolmasse von 15.500 g/mol (SternPEG-MMP). Die Hydrogele wurden durch Mischung der Ausgangsstoffe in einem molaren Verhältnis von 0,75 mol SternPEG-MMP zu 1 mol HEP-HM6, was einem Vernetzungsgrad von 0,75 entspricht, bei einem Gesamtfeststoffgehalt von 3,9%, gebildet. Hierfür wurden für jedes Hydrogel zuerst die Zellen in 5 Mikroliter (µl) PBS resuspendiert, dann wurden 5 µl HEP-HM6-Lösung (0,448 mg HEP-HM6 gelöst in 5 µl PBS) und 10 µl der SternPEG-MMP-Lösung (0,347 mg SternPEG-MMP gelöst in 10 µl PBS) dazugegeben, wie bei Tsurkan et al., Advanced Materials 2013, vol. 25 (18) pp. 2606-2610**,** beschrieben, innerhalb weniger Sekunden intensiv durchmischt und somit ein Endvolumen von 20 µl Hydrogel mit einer Konzentration an Zellen von 2x10⁶ Zellen/ml erzeugt. Die 20 µl Tropfen wurden sofort im Anschluss auf ein Parafilm-Blatt gebracht und weitere zwei Minuten gewartet, bis die Gelbildung vollständig erfolgt war. Die Gele wurden im Anschluss in 24-Well-Kultivierungsplatten gegeben, wobei jedes Well einen 20 µl-Tropfen Hydrogel und 1 ml an Volumen des Kulturmediums enthält. Die Hydrogele wurden dann bis zum gewünschten Zeitpunkt bei 5 % CO2 /95 % Luft bei 37 °C in den Wells kultiviert. Die resultierenden Hydrogele wiesen nach der Gelbildung ein Speichermodul in einem Bereich von 450±150 Pa auf, welches mittels oszillatorischer Rheometrie von in PBS bei Raumtemperatur gequollenen Hydrogelscheiben durch Nutzung eines Rotationsrheometers (ARES LN2; TA Instruments, Eschborn, Germany) mit einer Platte-Platte-Messanordnung bei einem Plattendurchmesser von 25 mm durch frequenzabhängige Messung bei 25 °C in einem Scherfrequenzbereich 10⁻¹ - 10² rad s⁻¹ mit einer Deformationsamplitude von 2% bestimmt wurde.

### Beispiel 2

Bildung von PEG-Heparin und Einbetten von Zellen:
Die PEG-Heparin-Gele wurden hergestellt, wie bei Tsurkan et al., Advanced Materials 2013, vol. 25 (18) pp. 2606-2610**,** beschrieben, mit folgenden Änderungen:
PHCC der zweiten Passage wurden dem Kulturgefäß unter Anwendung von Accutase® (Firma Invitrogen) als Zellablösungsmedium aus Kulturgefäßen entnommen. Nach der Zentrifugation für 10 min bei 12.000 Umdrehungen pro Minute wurden die PHCC bei einer Konzentration von 8x10⁶ Zellen pro ml in phosphatgepufferter Salzlösung (PBS) resuspendiert. Die polymeren Ausgangsstoffe für die Hydrogelherstellung bestanden, wie bei Tsurkan et al., Advanced Materials 2013, vol. 25 (18) pp. 2606-2610**,** beschrieben, aus Heparin, funktionalisiert mit sechs Maleimidgruppen (HEP-HM6) mit einem Molekulargewicht von 15.000 g/mol, und vierarmigem SternPEG, funktionalisiert mit enzymatisch spaltbaren Peptidsequenzen an jedem Arm mit einer Gesamtmolmasse von 15.500 g/mol (SternPEG-MMP). Die Hydrogele wurden durch Mischung der Ausgangsstoffe in einem molaren Verhältnis von 0,75 mol SternPEG-MMP zu 1 mol HEP-HM6 (entspricht einem Vernetzungsgrad von 0,75) bei einem Gesamtfeststoffgehalt von 3,9% gebildet. Hierfür wurden für jedes Hydrogel mit einem Gesamtvolumen von 20 µl zuerst die Zellen in 5 Mikroliter (µl) PBS resuspendiert, dann wurden 5 µl HEP-HM6-Lösung (0,448 mg HEP-HM6 gelöst in 5 µl PBS) und 10 µl der SternPEG-MMP -Lösung (0,347 mg SternPEG-MMP gelöst in 10 µl PBS) dazugegeben, wie bei Tsurkan et al., Advanced Materials 2013, vol. 25 (18) pp. 2606-2610**,** beschrieben, innerhalb weniger Sekunden intensiv durchmischt und somit ein Endvolumen von 20 µl Hydrogel mit einer Konzentration an Zellen von 2x10⁶ Zellen/ml erzeugt. Die 20 µl Tropfen wurden sofort im Anschluss auf ein Parafilm-Blatt gebracht und weitere 2 Minuten gewartet, bis die Gelbildung vollständig erfolgt war. Die Gele wurden im Anschluss in 24-Well-Kultivierungsplatten gegeben, wobei jedes Well einen 20 µl-Tropfen Hydrogel und 1 ml an Volumen des Kulturmediums enthält. Als Kulturbedingungen wurden 5 % CO2 /95 % Luft bei 37 °C angewendet. Die Hydrogele wurden dann bis zum gewünschten Zeitpunkt in den Wells kultiviert. Die resultierenden Hydrogele wiesen nach der Gelbildung ein Speichermodul in einem Bereich von 450±150 Pa auf, welches mittels oszilatorischer Rheometrie von in PBS bei Raumtemperatur gequollenen Hydrogelscheiben durch Nutzung eines Rotationsrheometers (ARES LN2; TA Instruments, Eschborn, Germany) mit einer Platte-Platte Messanordnung bei einem Plattendurchmesser von 25 mm durch frequenzabhängige Messung bei 25 °C in einem Scherfrequenzbereich 10⁻¹ - 10² rad s⁻¹ mit einer Deformationsamplitude von 2% bestimmt wurde.

Um Gele zu verwenden, die mit Amyloid-β-42 (Aß42) vorbehandelt wurden, wurden die Zellen vor der Zellentnahme aus dem Kulturgefäß und vor dem Einbetten der Zellen in das Hydrogel 48 Stunden lang mit 2 µM von Aß42 inkubiert. Um eine Gelumgebung zu erzeugen, die Aß42 enthält, wurden die Zellen zuerst in 4 µl von 100 µM Aß42-Peptid in PBS gelöst. 6 µl der HeparinLösung (0,448 mg HEP-HM6 gelöst in 6 µl PBS) und 10 µl der SternPEG-MMP-Lösung (0,347 mg SternPEG-MMP gelöst in 10 µl PBS) dazugegeben und, wie zuvor beschrieben, gemischt. In dieser Gelmischung beträgt die Konzentration von Aβ42 20 µM und die Konzentration der Zellen 2x10⁶ Zellen pro ml.

### Beispiel 3

Um Gele zu verwenden, die mit Amyloid-β-42 (Aß42) vorbehandelt wurden, wurden die Zellen vor der Zellentnahme aus dem Kulturgefäß und vor dem Einbetten der Zellen in das Hydrogel 48 Stunden lang mit 2 µM von Aß42 inkubiert.

### Immunozytochemie:

Alle Hydrogele wurden mit eisgekühltem Paraformaldehyd fixiert und 1,5 h bei Raumtemperatur inkubiert, gefolgt von einem Waschen in PBS über Nacht bei 4 °C. Für die Immunozytochemie wurden die Hydrogele in Blockierungslösung, die aus 10 % normalen Ziegenserum, 1 % Rinderserumalbumin, 0,1 % Triton-X in PBS bestand, 4 h lang über Nacht blockiert. Die Gele wurden für zwei aufeinanderfolgende Tage bei gelegentlichem Wechsel des PBS bei 4 °C gewaschen. Nach dem Waschen wurden die Gele mit sekundärem Antikörper bei Raumtemperatur für 6 Stunden inkubiert (1:500 in Blockierungslösung). Nach 3 Waschschritten von 2 Stunden wurde jeweils eine DAPI-Färbung durchgeführt (1:3000 in PBS, 2 Stunden bei Raumtemperatur).

### Fluoreszenzaufnahmen

Für die Hydrogele wurden Fluoreszenz-Aufnahmen unter Verwendung eines inversen Konfokal- und Multiphotonenmikroskops vom Typ Leica-SP5 durchgeführt. Die Hydrogele wurden in Petrischalen mit Glasboden platziert. 60 µl PBS wurden auf die Oberseite der Hydrogele gegeben, um ein Austrocknen zu verhindern. Die Z-Stapel wurden unter Verwendung einer Wassertauchlinse (25 x) eingefangen. Jeder Z-Stapel hat einen z-Abstand von 500 µm.

### Vergleich der Entwicklung von primären menschlichen kortikalen (PHCCs) und von iPSCs-abgeleiteten neuronalen Stamm- und Vorläuferzellen (NSPCs) in Stern-PEG-Heparin-Hydrogelen

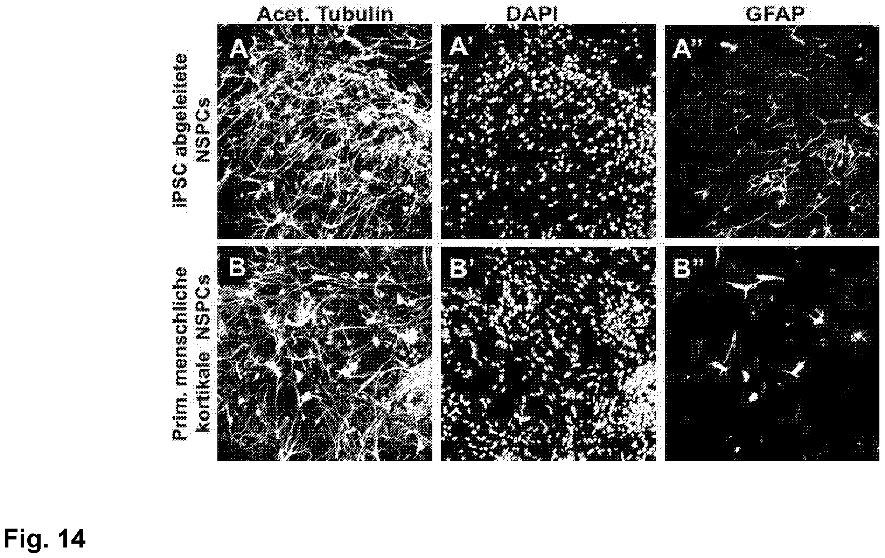

Die **Fig. 14** zeigt mikroskopische Aufnahmen, die einen Vergleich eingebetteter PHCCs und von iPSC-abgeleiteten NSPCs auf ihre Fähigkeit zur Bildung neuronaler Netzwerke in Stern-PEG-Heparin-Hydrogelen ermöglicht. Dabei zeigen die Abbildungen A-A" die maximale Intensitätsprojektion eines 500 µm dicken Z-Stapels von iPSC-abgeleiteten NSPCs, die in Stern-PEG-Heparin-Hydrogelen eingebettet wurden, welche mit RGD-Peptiden modifiziert wurden, gefärbt für Acetyliertes Tubulin (Acet. Tubulin, siehe Abbildung A), gefärbt mit DAPI, (Abbildung A') und gefärbt mit GFAP, (Abbildung A"). Die Abbildungen B-B" zeigen die maximale Intensitätsprojektion eines 500 µm dicken Z-Stapels von PHCCs, eingebettet in Stern-PEG-Heparin-Hydrogelen, gefärbt für Acetyliertes Tubulin, (Abbildung B), gefärbt mit DAPI (Abbildung B'), und gefärbt mit GFAP Antikörpern (Abbildung B").

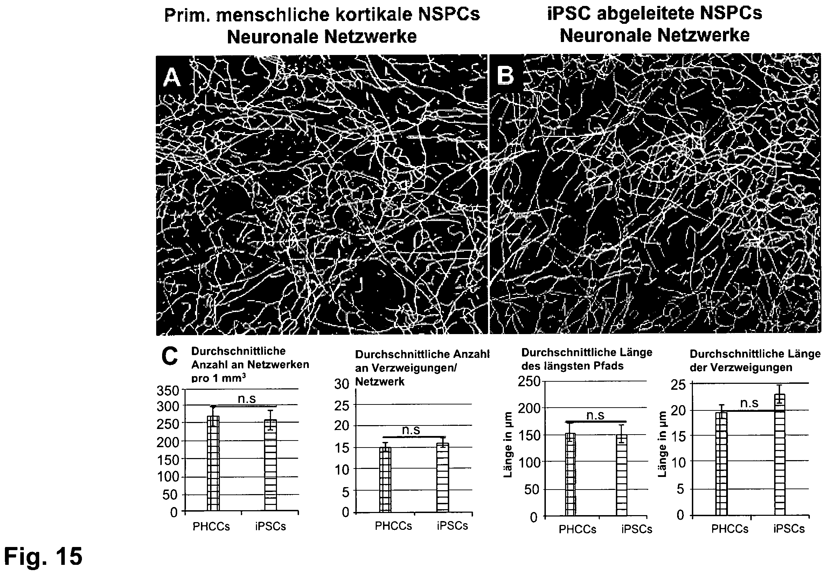

Die **Fig. 15** zeigt in Abbildung A die maximale Intensitätsprojektion der neuronalen Forstätze menschlicher kortikaler NSPCs nach TUBB3-Färbung. Die Abbildung B der **Fig. 15** zeigt die maximale Intensitätsprojektion der neuronalen Forstätze von iPSC abgeleiteten NSPCs nach TUBB3-Färbung. Die Abbildung C zeigt die Quantifizierung und Gegenüberstellung der neuronalen Netzwerkeigenschaften der Abbildungen A und B in Diagrammen.

Die gemäß der vorliegenden Erfindung verwendeten Hydrogele können kovalent mit verschiedenen Matrix-abgeleiteten Peptiden wie RGD (Arg-Gly-Asp) modifiziert oder für die effektive Verabreichung von löslichen Signalmolekülen verwendet werden. Auf diese Weise lassen sich Effekte von exogenen Signalen individuell auf die menschliche neuronale Stamm- und Vorläuferzellproliferation und die neuronale Netzwerkbildung testen. Die Tatsache, dass sich dieses Stern-PEG-Heparin-Hydrogelsystem mit einer Vielzahl von unterschiedlichen Molekülen modifizieren lässt, gibt einem Anwender die Möglichkeit, maßgeschneiderte Umgebungen zu schaffen. Beispielsweise ermöglicht es die Anpassung des PEG-HEP-Gerüstes mit RGD-Peptiden, menschliche iPSCs-abgeleitete neuronale Stamm- und Vorläuferzellen (NSPCs) zu kultivieren, wie in der **Fig. 14** gezeigt. Eine solche Methode ist ohne die RGD-Modifikation nicht möglich. Wie aus der **Fig. 15** hervorgeht, zeigen sich beim Vergleich der Fähigkeit der primären menschlichen kortikalen (PHCCs) einerseits und von iPSCs-abgeleiteten neuronalen Stamm- und Vorläuferzellen (NSPCs) andererseits, neuronale Netzwerke auszubilden, im verwendeten Hydrogelsystem keine Unterschiede. Die Anzahl an Netzwerken sowie die Anzahl und Länge der Verzweigungen ist vergleichbar, wie insbesondere die Abbildung C in **Fig. 15** verdeutlicht. Die sehr ähnliche Entwicklung von humanen iPSC-abgeleiteten neuronalen Stamm- und Vorläuferzellen (NSPCs) gegenüber denen aus primären menschlichen kortikalen Zellen (PHCCs) zeigt, dass das oben genannte Hydrogelsystem in einem breiten Anwendungsspektrum eingesetzt werden kann. Die vielversprechendsten Anwendungen des Hydrogelsystems sind auf dem Gebiet der personalisierten Medizin zu erwarten. Dafür sprechen insbesondere die Modifizierbarkeit, die Ansprechbarkeit auf Behandlungen, wie zum Beispiel mit Interleukin-4 (IL-4), sowie die Produzierbarkeit von großen Mengen der Stern-PEG-Heparin-Hydrogele in relativ kurzer Zeit. Durch die Kultivierung von iPSC-abgeleiteten Neuronen von Patienten ist es möglich, verschiedene neuronale Entwicklungsstörungen besser zu verstehen. Die generierten Hydrogele lassen sich aber auch zur Identifizierung von Behandlungsstrategien nutzen.

### Herstellung von PEG-Heparin-Gelen mit von iPSC-abgeleiteten neuralen Stamm- und Vorläuferzellen (NSPCs)

Humane neuronale Stamm- und Vorläuferzellen (NSPCs) abgeleitet aus iPSCs mit der Bezeichnung HIP™ (BC1 Linie) wurden von Amsbio (Katalog-Nummer: GSC-4311) bezogen. Diese NSCs wurden entsprechend der Herstellerangaben aufgetaut und in Geltrex-beschichteten Zellkulturflaschen kultiviert. Für die Expansion und die weitere Kultivierung der Zellen wurde das Expansionsmedium gemäß den Anweisungen des Herstellers verwendet. NeuralX™-NSC-Medium hat die folgende Zusammensetzung: 2% GS22™ Neuronal Supplement, 10; 1x Nicht-essentielle Aminosäuren, 2 mM L-Alanin / L-Glutamin; 20 ng / ml FGF2. Die HIP™-NSCs wurden mit Accutase (Invitrogen) aus den Zellkulturflaschen gelöst. Nach Zentrifugation bei 12.000 rpm für 10 Minuten wurden die HIP™-NSCs in PBS in einer Dichte von 8 x 10⁶ Zellen/ml resuspendiert. Für jedes Hydrogel wurden die Zellen zuerst in 5 Mikroliter (µl) PBS resuspendiert, dann wurden 5 µl Heparinlösung (45 µg/µl in PBS und 2M Integrin-Liganden als RGD-Peptide **(Tsurkan, Chwalek et al., 2011, Maitz, Freudenberg et al., 2013, Tsurkan** , **Chwalek et al. 2013, Wieduwild, Tsurkan et al. 2013)** durch gründliches Vortexen in PBS gelöst und 10 µl PEG dazugegeben, zu einem Endvolumen von 20 µl mit 2x10⁶ Zellen/ml. Es wurde ein 20 µl Tropfen auf ein Parafilm-Blatt aufgebracht. Die Gelbildung dauerte zwei Minuten. Die Gele wurden in 24-Well-Kultivierungsplatten gegeben, wobei jede Vertiefung 1 ml an Volumen des HIP-Expansionsmediums enthält. Zur Kultivierung der Gele wurden 5 % CO₂ /95 % Luft bei 37 °C angewendet. Die Gele können dann bis zum gewünschten Zeitpunkt kultiviert werden.

### Verwendung der PEG-HEP-Hydrogele zur Analyse des Effekts einzelner Faktoren auf die neurogene Plastizität und die Aß42-vermittelte Toxizität

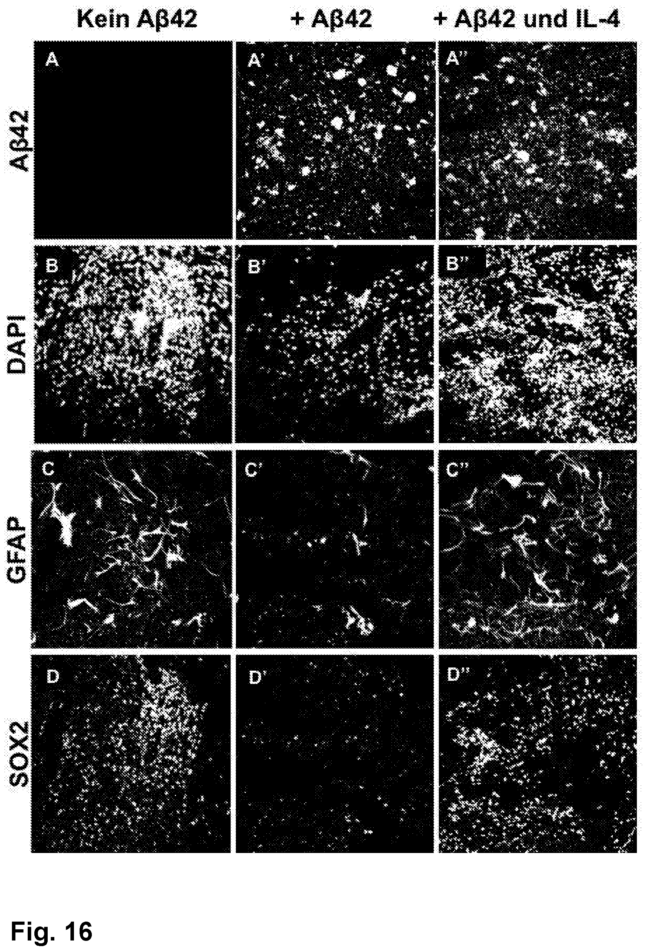

Die **Fig. 16** zeigt mikroskopische Aufnahmen von Stern-PEG-HEP-Gelen mit eingebetteten PHCCs aus der Kontrollgruppe ohne Aß42 (A-D), der Kontrollgruppe mit Aß42 (A'-D') und der Kultur mit Aß42 und Interleukin-4 (IL-4) (A"-D"), jeweils nach Färbung mit Anti-Aß42-Antikörpern (Abbildungen A-A"), nach Färbung mit DAPI (Abbildungen B-B"), mit Anti-GFAP-Antikörpern (Abbildungen C-C"), und nach Färbung mit Anti-SOX2-Antikörpern (Abbildungen D-D").

Um zu testen, ob IL-4 beim Menschen ähnlich wirkt, wie es zuvor zum Beispiel in Zebrafisch-Untersuchungen gezeigt werden konnte, wurden die oben genannten Stern-PEG-HEP-Hydrogele mit PHCCs verwendet. Hierzu wurden Hydrogele mit eingebetteten PHCCs hergestellt und diese mit Aß42 inkubiert, wie bereits beschrieben. Der Versuchsaufbau enthielt je eine positive (mit Aß42) und eine negative Kontrollgruppe (ohne Aß42) sowie eine experimentelle Gruppe mit Aß42 und IL-4. In der experimentellen Gruppe wurden die Stern-PEG-HEP-Gele mit eingebetteten PHCCs mit Aß42 und gleichzeitig in Anwesenheit von 100 ng / ml IL-4 im Medium kultiviert. Nach dreiwöchiger Kultivierungsphase wurden die Proben fixiert und immunologisch angefärbt gegen GFAP und SOX2, um Effekte von IL-4 auf die neuralen Stamm- und Vorläuferzellen zu untersuchen. Der Kernfarbstoff DAPI wurde verwendet, um sämtliche Zellen darzustellen. In mit Aß42 behandelten Zellkulturen war ein starker Rückgang der Zellzahl im Vergleich zu unbehandelten Kontrollkulturen zu beobachten, wobei sowohl GFAP-positive Gliazellen als auch SOX2-positive Neurone betroffen waren. In mit Aß42 und gleichzeitig mit IL-4 behandelten Kulturen war insgesamt die Zellzahl mit den Kontrollkulturen ohne Aß42-Behandlung vergleichbar. Die Ergebnisse deuten darauf hin, dass eine Behandlung mit IL-4 dem neurotoxischen Effekt von Aß42 entgegenwirkten kann. Es lässt sich schlussfolgern, dass die Behandlung mit Interleukin-4 GFAP-positive Zellen zur Proliferation anregt, mehr neuronale Stamm- und Vorläuferzellen zu bilden. IL-4 erhöht somit die Neuroplastizität der eingebetteten PHCCs trotz der Anwesenheit von neurotoxischem Aß42. Insgesamt zeigen die Daten, dass die Behandlung mit IL-4 die Proliferation von menschlichen neuronalen Stammzellen aktiviert, ähnlich wie im Zebrafischmodell gezeigt. IL-4 ist somit ein wichtiger Kandidat für zukünftige Therapien gegen Aß42 vermittelte Neurodegeneration. Die Gabe von spezifischen Aß42-Peptiden mit zellpenetrierenden Sequenzen in die vorliegenden Hydrogel-basierten 3D-Kulturen zeigt, dass die hier angewendete Zellkulturmethode die Pathophysiologie der menschlichen Aß42-Toxizität reproduzieren kann und dass im Stern-PEG-HEP-Hydrogel-System auch neuroprotektive Effekte untersucht werden können.

### Vergleich einer konventionellen Methode zur Herstellung von 3D Zellkulturen mit dem Stern-PEG-HEP-Hydrogel-System

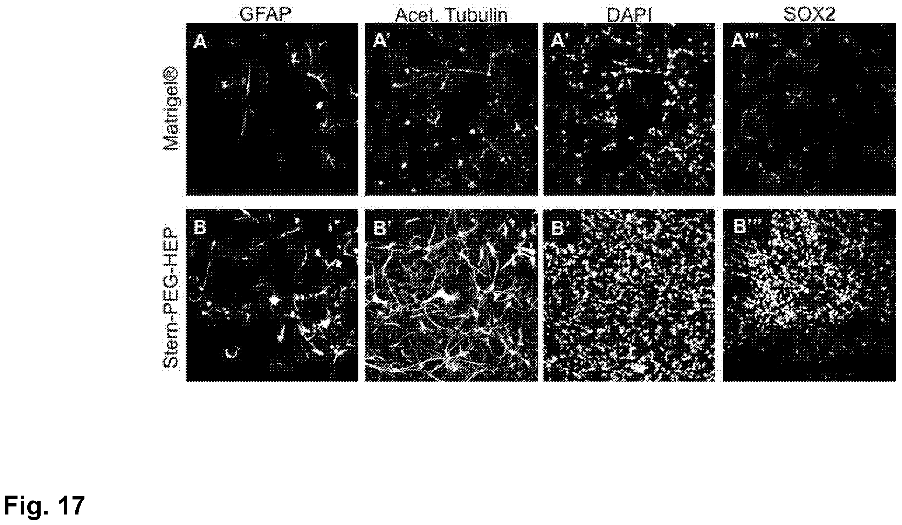

Die **Fig. 17** zeigt mikroskopische Aufnahmen nach Immunfärbung gegen GFAP, SOX2 und Acetyliertes Tubulin zum Vergleich von Matrigel- und Stern-PEG-Heparin-Hydrogelen, in die jeweils primäre humane kortikale Zellen (PHCCs) eingebettet sind. Dabei zeigen die Abbildungen A-A" PHCCs, welche in Matrigel eingebettet sind. Dagegen zeigen die Abbildungen B-B'" in Stern-PEG-Heparin-Hydrogele eingebettete PHCCs. Die Abbildungen A und B sind jeweils für saures Gliafaserprotein (GFAP) gefärbt, um die gliale Zellpopulation zu identifizieren; Die Abbildungen A' und B' sind jeweils gegen acetyliertes Tubulin (Acet Tubulin) gefärbt, um die neuronale Netzwerkbildung darzustellen. Die Abbildungen A" und B" enthalten eine DAPI-Färbung, um sämtliche Zellen zu markieren. Schließlich ermöglicht die Gegenüberstellung der Abbildungen A'" und B'" einen Vergleich des Ausmaß der Stammzellpopulationen und der neuroplastischen Kapazität mittels SOX2-Färbung.

Die dreidimensionale topologische Organisation im Organismus verleiht Geweben Eigenschaften wie Struktur, Abstammungsspezifikation und räumliche Wechselwirkung, die in herkömmlichen zweidimensionalen (2D)-Zellkulturen nicht reproduziert werden können. Hierdurch haben dreidimensionale (3D)-Zellkultursysteme einen deutlichen Vorteil und werden ausgiebig genutzt. Matrigel-basierte 3D-Zellkulturen stellen derzeit den bevorzugten Standard solcher Techniken dar, wobei neuronale Zellen in einem viskosen Gelmaterial, in die extrazelluläre Matrix (EZM)-Proteine, wie Kollagen und Laminin eingebettet sind, wachsen. Allerdings sind auf Matrigel-basierende Produkte chemisch undefiniert und heterogen in ihrer Zusammensetzung und lassen sich in verschiedenen Eigenschaften wie Steifheit, Gerüstzusammensetzung oder biologischer Ansprechbarkeit nicht verändern. Dies erschwert die Interpretation von Ergebnissen und es ist kaum möglich, die Einflüsse verschiedener exogener und parakriner Signale auf die zelluläre Entwicklung präzise zu analysieren. Das erfindungsgemäß verwendete Hydrogelsystem auf Basis von Heparin und PEG bietet dagegen wertvolle Vorteile, indem es die unabhängige Anpassung biophysikalischer und biomolekularer Matrixsignale ermöglicht. In einem direkten Vergleich zwischen dem Matrigelsystem und dem erfindungsgemäß verwendeten in **Fig. 17** ist leicht zu beobachten, dass die zelluläre Zusammensetzung der Gliazellen in beiden Matrixsystemen sehr ähnlich ist, aber die neurogene Fähigkeit und die Fähigkeit der menschlichen Stammzellen zur Bildung von neuronalen Netzwerken in den Stern-PEG-Heparin-Hydrogelen deutlich höher ist als in der Matrigel-Matrix. An dieser Stelle sei darauf hingwiesen, dass beide Kultursysteme für den gleichen Zeitraum von drei Wochen im gleichen Wachstumsmedium ohne weitere Zusätze kultiviert wurden. Die Beobachtung neuronaler Netzwerke im Stern-PEG-Heparin-Hydrogel, aber nicht im Matrigelsystem wird bestätigt durch die Tatsache, dass im Matrigelsystem kaum reife synaptische Verbindungen zwischen Neuronen identifiziert werden konnten. Im Gegensatz hierzu bildeten die Neurone in den Stern-PEG-Heparin-Hydrogelen neuronale Netzwerke aus. Der Grund, dass NSPCs im Matrigelsystem ihre neurogenen Eigenschaften nicht manifestieren und keine Netzwerke bilden, liegt vermutlich an dem hochgradig unorganisierten EZM-Milieu, das ähnlich wie Narbengewebe wirkt.

### Herstellung der Matrigel-3D Kultur

Für die Matrigel-Zellkulturen wurde Matrigel der Firma BD Biosciences (Katalognummer: 356234) verwendet. Vor jeder Zellkulturarbeit und Verwendung von Matrigel wurden Pipettenspitzen und Eppendorf Tubes entsprechend der Herstelleranweisung zur "Dick-Gelmethode" bei -20 °C eingefroren. Das Matrigel wurde bei 4°C über Nacht auf Eis aufgetaut. PHCCs der zweiten Passage wurden aus Zellkulturflaschen unter Verwendung von Accutase (Invitrogen) gelöst. Nach einer Zentrifugation (bei 12.000 rpm für 10 Minuten) wurden die PHCCs in BD Matrigel in einer Dichte von 2x10⁶ Zellen pro ml resuspendiert. Tröpfchen der Zell-/Matrigel-Mischung wurden erzeugt, um bei 37°C zu erstarren. Anschließend wurde Zellkulturmedium (SRL, Katalognummer 1801) zugegeben und die Gele für drei Wochen kultiviert. Dabei wurde das Zellkulturmedium am Tag nach der Herstellung der Zell-/Matrigel-Mischung und anschließend an jedem zweiten Tag gewechselt.

### Abkürzungsliste

- Aβ42: Amyloid-β-42
- Acet.Tub: acetyliertes Tubulin
- aTub: acetyliertes Tubulin
- BrdU: Bromodesoxyuridin
- CTIP2: ein Markerprotein für reife Kortikalneuronen, auch bekannt unter der englischen Bezeichnung. "B-cell CLL/lymphoma 11B", BCL11b
- DAPI: 4',6-Diamidin-2-phenylindol, ein Zellkernfarbstoff
- GFAP: saures Gliafaserprotein, englisch "Glial fibrillary acidic protein", zytoplasmatischer Marker für Gliale Zellen
- Gcamp: Calciumsensor
- HEP-HM6: Heparin, konjugiert mit sechs Maleimidgruppen
- IL-4: Interleukin-4
- iPSCs: induzierte pluripotente Stammzellen
- MMP: Matrixmetalloprotease
- NSPCs: Humane neuronale Stamm- und Vorläuferzellen; Abkürzung des englischen Terminus: neural stem and progenitor cells
- PBS: phosphatgepufferte Salzlösung
- PEG: Polyethylenglykol
- PHCC: primäre humane kortikale Zellen; Abkürzung des englischen Terminus: primary human cortical cells
- HEP: Heparin
- SATB2: ein Markerprotein für reife Kortikalneuronen, Abkürzung für die englische Bezeichnung "Special AT-rich sequence-binding protein 2"
- SOX2: Transkriptionsfaktor, Abkürzung für "geschlechtsbestimmende Region Y- Box 2"
- SternPEG- MMP: sternförmiges (vierarmiges) Polyethylenglykol, endfunktionalisiert mit enzymatisch (Matrixmetalloprotease-) spaltbaren Peptid-Linkern
- Syn: Synaptophysin
- TUBB3: Beta-III-Tubulin, neuronaler zytoplasmatischer Marker

## Patentansprüche

1. Verfahren zur Bildung eines funktionellen Netzwerkes von humanen neuronalen und glialen Zellen, bei dem die Zellen in ein synthetisches Hydrogelsystem mit den Komponenten Polyethylenglykol (PEG) und Heparin eingebracht und darin kultiviert werden, wobei das Einbringen der Zellen in das PEG-Heparin-Hydrogelsystem zusammen mit einer der Gelkomponenten, entweder PEG oder Heparin, erfolgt, mit der die Zellen zuvor vermischt wurden, so dass sich die Zellen während der Bildung des dreidimensionalen Hydrogels bereits im Hydrogelsystem befinden.

2. Verfahren nach Anspruch 1, **dadurch gekennzeichnet, dass** die humanen neuronalen Zellen mit glialen Zellen co-kultiviert werden und dass die humanen neuronalen Zellen humane neuronale Stamm- und Vorläuferzellen sind oder einer humanen immortalisierten neuronalen Vorläuferzelllinie entstammen oder primäre humane, aus dem Mittelhirn gewonnene neuronale Vorläuferzellen sind.

3. Verfahren nach Anspruch 2, **dadurch gekennzeichnet, dass** die humanen neuronalen Zellen humane neuronale Stamm- und Vorläuferzellen aus induzierten pluripotenten Stammzellen (iPSCs) sind oder aus primären humanen kortikalen Zellen abgeleitet sind.

4. Verfahren nach einem der Ansprüche 1-3, **dadurch gekennzeichnet, dass** die Funktionalität durch die Expression reifer neuronaler kortikaler Marker, die Ansprechbarkeit auf Neurotransmitter und durch elektrophysiologische Aktivität beschreibbar ist.

5. Verfahren nach einem der Ansprüche 1 bis 4, **dadurch gekennzeichnet, dass** das PEG-Heparin-Hydrogelsystem ein ein mehrarmiges Polyethylenglykol (Stern-PEG) enthaltendes Stern-PEG-Heparin-Hydrogelsystem ist, das über enzymatisch spaltbare Peptidsequenzen vernetzt wird, wodurch das Stern-PEG-Heparin-Hydrogelsystem spaltbar und lokal umbaubar ist.

6. Verfahren nach Anspruch 5, **dadurch gekennzeichnet, dass** die Hydrogelmatrix des Hydrogels durch eine kovalente Vernetzung eines thiolterminierten Stern-PEG-Peptid-Konjugats und eines durch Maleimid funktionalisierten Heparins gebildet wird, wobei die Vernetzung der Hydrogelmatrix über eine Michael-Addition erfolgt.

7. Verfahren nach Anspruch 5, **dadurch gekennzeichnet, dass** die Hydrogelmatrix des Stern-PEG-Heparin-Hydrogelsystems nichtkovalent aus Heparin und einem kovalenten Stern-PEG-Peptid-Konjugat durch Selbstorganisation gebildet wird, wobei das Stern-PEG-Peptid-Konjugat Konjugate von zwei oder mehr Peptiden umfasst, die an eine Polymerkette gekoppelt sind und die Peptidsequenz ein sich wiederholendes Dipeptid-Motiv (BA)ₙ enthält, wobei B eine Aminosäure mit einer positiv geladenen Seitenkette, A Alanin und n eine Zahl von 5 bis 20 ist.

8. Verfahren nach einem der Ansprüche 1 bis 7, **dadurch gekennzeichnet, dass** das Hydrogel variierbare mechanische Eigenschaften, charakterisiert durch das Speichermodul, vorzugsweise in einem Bereich von 300 - 600 Pascal, aufweist.

9. Verfahren nach einem der Ansprüche 1 bis 8, **dadurch gekennzeichnet, dass** das PEG-Heparin-Hydrogelsystem mit Signalmolekülen und/oder mit von Proteinen der extrazellulären Matrix (EZM) abgeleiteten funktionellen Peptideinheiten modifiziert wird.

10. Verfahren nach einem der Ansprüche 1 bis 9, **dadurch gekennzeichnet, dass** eine Co-Kultivierung der humanen neuronalen und glialen Zellen zusammen mit humanen mesenchymalen Stromazellen und Endothelzellen erfolgt, die colokalisiert mit den humanen neuronalen und glialen Zellen vorliegen.

11. Humanes, neuronales, dreidimensionales funktionelles Netzwerk, erhältlich durch ein Verfahren nach einem der Ansprüche 1 bis 10.

12. Anwendung des Verfahrens nach einem der Ansprüche 1 bis 10 zum Monitoring der Bildung des neuronalen Netzwerks.

13. Anwendung nach Anspruch 12 zum Echtzeit-Monitoring der Bildung des neuronalen Netzwerks.

14. Anwendung nach Anspruch 12 oder 13, **dadurch gekennzeichnet, dass** eine quantitative Analyse des Zellwachstums, der Länge, der Anzahl und Dichte von Verzweigungen und/oder der Konnektivität und/oder der elektrophysiologischen Aktivität der neuronalen Zellen innerhalb des neuronalen Netzwerkes erfolgt.

15. Anwendung nach einem der Ansprüche 12 bis 14 zur Modellierung von Krankheiten, die sich auf die Bildung von Neuronen und/oder neuronalen Netzwerken im menschlichen Gehirn auswirken.

16. Anwendung nach Anspruch 15 zur Modellierung der durch krankheitsrelevante Proteinaggregate verursachten Neurotoxizität und/oder Veränderung der neuronalen Stammzellplastizität.

17. Anwendung nach einem der Ansprüche 12 bis 16 für den Test von Molekülen und/oder Wirkstoffen, die die neuronale Aktivität und/oder Netzwerkbildung beeinflussen.

## Claims

1. Process for formation of a functional network of human neuronal and glial cells, in which the cells are introduced into and cultivated in a synthetic hydrogel system with the components polyethylene glycol (PEG) and heparin, wherein the cells are introduced into the PEG-heparin-hydrogel system together with a gel component, either PEG or heparin, with which the cells were also previously mixed, so that the cells are already located in the hydrogel system during formation of the three-dimensional hydrogel.

2. Process according to claim 1, **characterised in that** the human neuronal cells are co-cultivated with glial cells and that the human neuronal cells are human neuronal stem and progenitor cells or originate from a human immortalised neuronal line of progenitor cells or are primary human neuronal progenitor cells extracted from the midbrain.

3. Process according to claim 2, **characterised in that** the human neuronal cells are human neuronal stem and progenitor cells from induced pluripotent stem cells (iPSCs) or are derived from primary human cortical cells.

4. Process according to one of the claims 1-3, **characterised in that** the functionality can be described through expression of mature neuronal cortical markers, the response to neurotransmitters and through electrophysiological activity.

5. Process according to one of the claims 1 to 4, **characterised in that** the PEG-heparin-hydrogel system is a star-PEG-heparin-hydrogel system that contains a multi-arm polyethylene glycol (star-PEG) which is crosslinked via enzymatically cleavable peptide sequences, meaning that the star-PEG-heparin-hydrogel system is cleavable and locally modifiable.

6. Process according to claim 5, **characterised in that** the hydrogel matrix of the hydrogel is formed through a covalent crosslinking of a thiol-terminated star-PEG-peptide conjugate and of a heparin functionalised by maleimide, wherein the crosslinking of the hydrogel matrix is performed via a Michael reaction.

7. Process according to claim 5, **characterised in that** the hydrogel matrix of the star-PEG-heparin-hydrogel system is formed non-covalently from heparin and a covalent star-PEG-peptide conjugate via self-organisation, wherein the star-PEG-peptide conjugate encompasses conjugates from two or more peptides which are linked to a polymer chain and the peptide sequence contains a repeating dipeptide motif (BA)ₙ, wherein B is an amino acid with a positively charged side chain, A is alanine and n represents a number from 5 to 20.

8. Process according to one of the claims 1 to 7, **characterised in that** the hydrogel exhibits variable mechanical properties, **characterised by** the storage module, preferably in a range from 300 - 600 Pascal.

9. Process according to one of the claims 1 to 8, **characterised in that** the PEG-heparin-hydrogel system is modified with signal molecules and/or with functional peptide units derived from proteins of the extracellular matrix (ECM).

10. Process according to one of the claims 1 to 9, **characterised in that** a cocultivation of the human neuronal and glial cells is performed together with human mesenchymal stromal cells and endothelial cells, which are available in co-localised form with the human neuronal and glial cells.

11. Human, neuronal, three-dimensional functional network, available through a process according to one of the claims 1 to 10.

12. Application of the process according to one of the claims 1 to 10 for monitoring the formation of the neuronal network.

13. Application according to claim 12 for real-time monitoring of the formation of the neuronal network.

14. Application according to claim 12 or 13, **characterised in that** a quantitative analysis of the cell growth, the length, the number and density of branches and/or the connectivity and/or electrophysiological activity of the neuronal cells within the neuronal network is performed.

15. Application according to one of the claims 12 to 14 for modelling of diseases that have an impact on the formation of neurons and/or neuronal networks in the human brain.

16. Application according to claim 15 for modelling of the neurotoxicity caused by disease-related protein aggregates and/or the change in neuronal stem cell plasticity.

17. Application according to one of the claims 12 to 16 for the test of molecules and/or active substances that influence neuronal activities and/or network formation.

## Revendications

1. Procédé destiné à former un réseau fonctionnel de cellules neuronales et gliales humaines, consistant à introduire les cellules dans un système d'hydrogel synthétique comprenant les composants suivants polyéthylène glycol (PEG) et héparine, et à les y cultiver dedans, sachant que l'introduction des cellules dans le système d'hydrogel PEG-héparine a lieu avec l'un des composants du gel, soit le PEG soit l'héparine, composant avec lequel les cellules ont préalablement été mélangées, de sorte que les cellules se trouvent déjà dans le système d'hydrogel pendant la formation de l'hydrogel tridimensionnel.

2. Procédé selon la revendication 1, **caractérisé en ce que** les cellules neuronales humaines sont co-cultivées avec des cellules gliales et que les cellules neuronales humaines sont des cellules souches et cellules précurseures neuronales humaines, ou qu'elles proviennent d'une ligne de cellules précurseures neuronales humaines immortalisées, ou que ce sont des cellules précurseures neuronales, primaires humaines, obtenues à partir du mésencéphale.

3. Procédé selon la revendication 2, **caractérisé en ce que** les cellules neuronales humaines sont des cellules souches et des cellules précurseures neuronales humaines provenant de cellules souches pluripotentes induites (iPSC), ou qu'elles sont dérivées de cellules corticales humaines primaires.

4. Procédé selon l'une des revendications 1 à 3, **caractérisé en ce que** la fonctionnalité est descriptible par l'expression de marqueurs corticaux neuronaux mûrs, par la réactivité aux neurotransmetteurs et par l'activité électrophysiologique.

5. Procédé selon l'une des revendications 1 à 4, **caractérisé en ce que** le système d'hydrogel PEG-héparine est un système d'hydrogel PEG en étoile-héparine contenant un polyéthylène glycol multibranches (PEG en étoile), réseauté par des séquences peptidiques fissiles par la voie enzymatique, faisant que le système d'hydrogel composé d'héparine et de PEG en étoile est fissile et localement convertible.

6. Procédé selon la revendication 5, **caractérisé en ce que** la matrice de l'hydrogel est formée par une réticulation covalente d'un conjugué PEG en étoile-peptide à terminaison thiol, et d'une héparine fonctionnalisée par de la maléimide, sachant que la réticulation de la matrice d'hydrogel a lieu via une addition de Michael.

7. Procédé selon la revendication 5, **caractérisé en ce que** la matrice d'hydrogel du système d'hydrogel PEG en étoile-héparine est formée de manière non covalente d'héparine et d'un conjugué covalent de PEG en étoile-héparine par auto-organisation, sachant que le conjugué de PEG en étoile-héparine comprend des conjugués de deux ou plusieurs peptides couplés à une chaîne polymère, et que la séquence peptidique contient un motif dipeptidique (BA)ₙ se répétant, sachant que B est un acide aminé avec une chaîne latérale de charge positive, A est de l'alanine et n est un nombre compris entre 5 et 20.

8. Procédé selon l'une des revendications 1 à 7, **caractérisé en ce que** l'hydrogel présente des propriétés mécaniques qu'il est possible de faire varier, **caractérisées par** le module d'accumulation situé de préférence entre 300 et 600 Pascal.

9. Procédé selon l'une des revendications 1 à 8, **caractérisé en ce que** le système d'hydrogel PEG-héparine est modifié à l'aide de molécules de signal et/ou à l'aide d'unités peptidiques fonctionnelles dérivées de protéines de la matrice extracellulaire (ECM).

10. Procédé selon l'une des revendications 1 à 9, **caractérisé en ce qu'a** lieu une co-culture des cellules neuronales et gliales humaines avec des cellules stromales mésenchymales humaines et des cellules endothéliales, qui sont présentes colocalisées avec des cellules neuronales et gliales humaines.

11. Réseau fonctionnel neuronal humain, obtenu au moyen d'un procédé selon l'une des revendications 1 à 10.

12. Application du procédé selon l'une des revendications 1 à 10, pour surveiller la formation du réseau neuronal.

13. Application selon la revendication 12, pour surveiller en temps réel la formation du réseau neuronal.

14. Application selon la revendication 12 ou 13, **caractérisée en ce qu'**a lieu une analyse quantitative de la croissance cellulaire, de la longueur, du nombre et de la densité des ramifications et/ou de la connectivité et/ou de l'activité électrophysiologique des cellules neuronales à l'intérieur du réseau neuronal.

15. Application selon l'une des revendications 12 à 14, pour modéliser des maladies qui ont des répercussions sur la formation des neurones et/ou des réseaux neuronaux dans le cerveau humain.

16. Application selon la revendication 15, pour modéliser la neurotoxicité provoquée par des agrégats protéiques pertinents de la maladie et/ou de l'altération de la plasticité des cellules souches neuronales.

17. Application selon l'une des revendications 12 à 16 pour tester des molécules et/ou principes actifs qui influencent l'activité neuronale et/ou la formation de réseaux.
